# EUROPEAN PATENT SPECIFICATION

(11) **EP 3 781 028 B1**
(45) Date of publication and mention of the grant of the patent: **10.07.2024**
(21) Application number: 19719809.6
(22) Date of filing: 18.04.2019
(51) Int. Cl.: A61B 5/0205, A61B 5/087, G01F 1/44, G01F 1/68

(54) **PORTABLE HANDHELD ELECTRONIC SPIROMETER**
TRAGBARES ELEKTRONISCHES HAND-SPIROMETER
SPIROMÈTRE ÉLECTRONIQUE PORTATIF

(30) Priority: 19.04.2018 EP 18460022
(43) Date of publication of application: 24.02.2021
(73) Proprietor: Healthup S.A., 00-105 Warsaw (PL)
(72) Inventor: BAJTALA, Piotr, 03-810 Warsaw (PL); KOLTOWSKI, Lukasz, 03-704 Warsaw (PL)
(74) Representative: Synergy IP Group AG
(86) International application number: PCT/EP2019/060193
(87) International publication number: WO 2019/202126

(56) References cited:
- EP-A1- 0 552 916
- US-A- 6 032 527
- US-A1- 2002 026 937
- US-A1- 2004 039 295
- US-A1- 2004 186 390
- US-A1- 2010 305 466
- US-A1- 2011 066 042
- US-A1- 2013 172 773
- US-A1- 2013 217 979
- US-A1- 2013 267 864
- US-A1- 2016 256 073
- US-A1- 2018 008 790
- US-B1- 6 322 519
- US-B1- 9 861 299

## Description

### Background of the Invention

The invention is related to a portable, handheld electronic spirometry device, or spirometer, as well as a method for determining lung function parameters using said device, for instance during full spirometry as defined by the spirometry standards of the American Thoracic Society (ATS) and the European Respiratory Society (ERS).

Spirometry is one of the most common tests used for determining, or evaluating, pulmonary function in terms of lung function parameters relating to the amount (volume) and/or speed (flow, or flow rate) of air that can be inhaled and exhaled, either forcedly or under normal breathing. The primary signals measured in spirometry may be volume and/or flow. Results are provided both as raw data (litres, litres per second) and as percent predicted, i.e. in relation to predicted values for healthy individuals of similar parameters such as height, age, sex, weight and sometimes ethnicity. Since multiple publications of predicted values are available, the interpretation of the results may vary slightly, but generally speaking, results close to or above100 % predicted are the most normal, and results ≥ 80 % are usually also considered normal. Commonly, the results are further displayed as graphs, so called spirograms or pneumotachographs, showing a volume-time curve (volume in litres on the Y-axis and time in seconds on the X-axis) and/or a flow-volume loop (depicting the rate of airflow on the Y-axis and the total volume inhaled or exhaled on the X-axis).

Spirometry is an important tool in the assessment of various obstructive or restrictive lung conditions such as asthma, chronic obstructive pulmonary disease (COPD), bronchitis, emphysema, pulmonary fibrosis (PF), and also cystic fibrosis (CF), because the tests performed with the so-called spirometer (i.e. a device, or apparatus, for measuring ventilation, the movement of air into and out of the lungs) are able to identify abnormal ventilation patterns, namely obstructive and restrictive patterns.

Lung function parameters that can be determined using spirometry and/or a spirometer include e.g.: vital capacity (VC; volume exhaled after deepest inhalation); forced vital capacity (FVC; determination of the vital capacity from a maximally forced expiratory effort); slow vital capacity (SVC); forced expiratory flow (FEF), peak expiratory flow (PEF; highest forced expiratory flow, measured with a peak flow meter or spirometer); forced expiratory volume (FEVx; volume of air exhaled under forced conditions in the first X seconds; e.g. FEV₁ = volume force-exhaled within 1^{st} second); forced expiratory time (FET), inspiratory vital capacity (IVC; maximum volume inhaled after maximum expiration); forced inspiratory vital capacity (FIVC); tidal volume (TV; volume of air moved into or out of the lungs during quiet breathing); inspiratory or expiratory reserve volume (IRV and ERV; maximal volume that can be inhaled or respectively exhaled from the end-inspiratory level or respectively end-expiratory level); inspiratory capacity (IC; sum of IRV and TV); maximal voluntary ventilation (MW; also called maximum breathing capacity); and others.

As mentioned, the test procedures are performed using a spirometer. Various types of these devices are known, from simple mechanically operating to fully electronic ones; said devices using a number of different measurement principles such as water gauges, 'windmill'-type rotors (also called turbines) or pressure transducers. Most conventional spirometers evaluate the fluid flow by measuring either a pressure difference before and after a membrane, capillaries or other forms of flow restriction with a known resistance (e.g. using a differential pressure sensor), or by the rotations of a turbine. In the past, efforts increased to render the devices portable and/or handheld, in order to obtain a more detailed and concise monitoring of e.g. therapy efficacy by allowing the patients, or users, to perform spirometry measurements by themselves; thereby obviating the need to visit a doctor's office or a hospital. Some of these portable devices are even aimed at being connectable to e.g. a patient's smartphone.

For instance, the Vitalograph's asma-1 device is a small, handheld, AAA-battery-powered device to measure and store PEF and FEV₁ values. The device is equipped with a rotatable turbine and disposable mouthpieces and can be connected via USB or Bluetooth to a mobile phone, PDA, PC or home hub. Unfortunately, the device can store only a limited number of measurements (up to 600) and no parameters other than PEF and FEV₁ can be measured. In other words, no full spirometry as defined by the spirometry standards of the American Thoracic Society (ATS) and the European Respiratory Society can be performed by the device; see "Standardisation of spirometry"; Eur Respir J 2005; 26: 319-338 (for instance, these standards define 24 ATS waveforms that the spirometer must correctly identify, some of these generated at higher temperature of 37 °C and high humidity, and additionally, the total resistance to airflow at 0-14.0 L/s must be <0.15 kPa/(L/s)).

Medical International Research's (MIR) offers a broad range of devices for measurements of respiratory parameters, some of them portable and some connectable to mobile phones. For instance, the Smart One^{®} device is a portable turbine flowmeter, optionally using MIR's customary disposable FlowMIR^{®} turbine and cardboard mouthpiece. The device can be connected via Bluetooth to a smartphone on which the respective Smart One^{®} app (available for iOS and Android) and the measured respiratory data are stored. The device is capable of determining e.g. PEF and FEV₁; however, no full spirometry can be performed with the device.

MIR's Spirodoc^{®} and Spirobank^{®}II Smart devices are portable, pocket-sized, stand-alone (i.e. requiring no computer) turbine flowmeter devices capable of performing full spirometry and storing up to 10.000 spirometry tests. The Spirodoc^{®} device comprises an approximately palm-sized main body with an LCD touchscreen display, an attachable flowmeter head housing a bi-directional digital turbine (e.g. the disposable FlowMIR^{®} turbine), and a temperature sensor for BTPS conversion of FVC measurements (i.e. vital capacity at maximally forced expiratory effort, expressed in litres at body temperature and ambientpressure saturated with water vapour). The Spirobank^{®}II Smart device differs mainly in that a keyboard is used instead of Spirodoc^{®}'s touchscreen and in that the flowmeter head is permanently fixed. Alternatively to the keyboard, the Spirobank^{®}II Smart device may also be operated via a tablet computer (iPad^{™}). A smartphone connectivity is not provided, though.

Both devices may optionally further comprise a fingertip pulse oximeter that can be attached via cable to the main body as separate components. A built-in three-axis movement sensor is provided in the devices in order to correlate the oxygen saturation level (%SpO2) measured with the fingertip oximeter to the user's physical activity. Data transmission, e.g. to a personal computer (PC) running the related WinspiroPRO^{®} software - or for the Spirobank^{®} Smart an iPad/iPad mini running the iOS-based MIR Spiro^{®} app - may be achieved via Bluetooth or USB connection. Only when connected to a PC or iPad, the respective software allows for real time spirometry and oximetry tests; i.e. real time curve display. Unfortunately, this need of e.g. a tablet computer or the like increases the costs for these devices.

A further portable, pocket size homecare spirometer in the product range of MIR is the Spirotel^{®} which uses an attachable, reusable bi-directional digital turbine and a small touchscreen in a main-body that is connectable to a personal computer (PC) via a USB-cable or Bluetooth; a software application (WinspiroPro Home Care) then extracts the data and sends it to a server. Same as with the Spirodoc^{®} and Spirobank^{®}II Smart devices, the Spirotel^{®} may optionally further comprise a fingertip pulse oximeter that can be attached via cable to the main body, and a built-in three axis movement sensor to correlate the measured oxygen saturation level (%SpO2) to the user's physical activity. While being portable itself, the Spirotel^{®} device cannot be used as a standalone and is not connectable to smart phones but requires the use of a PC instead.

One common disadvantage of most of the above listed devices is the use of movable parts, namely the turbines or rotating wings, to measure gas flow. This necessitates regular external calibrations, e.g. annually or biannually. Furthermore, a majority also lacks the option to measure spirometric parameters such as FEV₆, SVC or MW. In addition, the accuracy of FVC-measurements is often limited, especially in patients with severe obstruction characterized by prolonged exhale with very low flows at the end of exhale.

A portable, battery-operated device using a gas flow sensor without movable parts is the SpiroTube mobile edition by Thor Laboratories, a pulmonary function diagnostics and monitoring device with Bluetooth or USB connection to a PC (storing the ThorSoft pulmonary diagnostics PC software). Bluetooth and also WIFI connection is available as an option to connect the SpiroTube to iPad/iPhone, Android smartphones, PDA devices as well as any JAVA-ready mobile device. The SpiroTube uses the proprietary IDEGEN^{™} multipath measurement principle wherein the flow volume measurement depends on the quantity and energy of gas molecules, measured using ultrasound and the Doppler Effect. The inner surface of the flow tube is continuous and free of any obstacles such that it can be disinfected easily. However, due to its high power consumption and its significant weigth and size, the device is not suited for use as a portable, handheld, or pocket-sized, device.

A further device without movable parts is the WING device by US-based Sparo Labs which can be cable-connected to a smartphone via the headphone jack and which measures PEF (peak expiratory flow) and FEV, (volume force-exhaled after 1 second). Measured data are synced to a 'cloud' in encrypted form and can be analysed using a dedicated smartphone application. Unfortunately, no parameters other than PEF and FEV₁ can be measured (e.g. no forced vital capacity (FVC), forced expiratory flow at 25 %-75 % of FVC (FEF25-75), etc.). In other words, no full spirometry as defined by the spirometry standards of the American Thoracic Society (ATS) and the European Respiratory Society can be performed; similar to e.g. the asma-1 device described above. Also, the WING runs on the phone's battery (via the headphone jack), such that it is at risk to not measure data properly if the phone battery is low.

Alternatively, acceleration sensors (also called accelerometers) such as MEMS-based thermal fluid flow sensors (MEMS; microelectromechanical systems) have also been suggested in the prior art for flow measurements in medical devices including ventilators, sleep apnoea devices, spirometers, etc. These MEMS-based thermal fluid flow sensors use temperature sensors, such as thermocouples, and gas molecules heated via a resistive heating element. When subjected to acceleration, the less dense molecules in the heated gas move in the direction of acceleration and the cool and denser molecules move in the opposite direction, creating an acceleration proportional temperature difference measured by the temperature sensors. However, to the best of the inventor's knowledge, this conceptual idea of employing MEMS-based thermal fluid flow sensors for flow measurements in medical devices has never been translated into an existing, operable, functional and most importantly portable, handheld spirometer before that is sensitive enough to enable full spirometry; i.e. up to the present invention, it was not clear whether the concept could actually be put into practice and how exactly accurate and reproducible, or precise, spirometric flow measurements could be achieved; for instance, for performing full spirometry.

One example of a medical device presumably using a MEMS-based thermal flow sensor or similary sensor type, is provided in EP 0552916 A1 which describes a ventilator system with a flow-meter, wherein the flow-meter may *inter alia* employ a miniaturized thermal bridge-type, linear mass flow sensor such as a mass airflow integrated circuit chip sensor (e.g. commercially available from Microswitch under the trademark MICROBRIDGE). Other similar types of flow sensors are said to also be suitable. While the flow-meter in EP 0552916 A1 is called `spirometer', this term is misleading in that a ventilator, or respirator, is described in this document; i.e. a system that is used with intubated human subjects requiring respiratory support. These subjects, or patients, are not breathing spontaneously and would thus not be able to perform diagnostic lung function parameter tests such as full spirometry including forced inspiratory and expiratory breath maneouvres. In other words, EP 0552916 A1 does not refer to 'spirometry' in the sense of the present invention but instead more generally to any sort of flow rate measurements of air flows to and from a subject's lung which are generated by the ventilator, not the intubated and ventilated human subject. In addition, EP 0552916 A1 teaches that the desired airflow range is to be measured up to 300 SLM; in contrast, spirometry in the sense of the present invention requires evaluation of e.g. forced exhalations with flow rates up to 840 SLM, in some instances even up to 1000 SLM, rendering the flow-meter set-up described in EP 0552916 A1 unsuitable for use in a portable, handheld electronic spirometer as targeted by the present invention. Furthermore, due to the use of the venturi-element, the device cannot differentiate between inhalation or exhalation flows.

US 6,322,519 B1 discloses a device for measuring exhaled flow with a differential pressure sensor.

It is an object of the present invention to provide an improved portable spirometer which overcomes the draw backs of prior art devices; e.g. a device with higher measurement sensitivity that can be used without medically trained staff and which is capable of performing full spirometry, including measurements of main spirometry paramters such as FEV₁, FVC, PEF, and FEV₁% but also parameters such as FEV₆, SVC or MW. This object is achieved by the subject matter of the present invention as set forth in the claims, namely by a portable spirometer employing MEMS based thermal fluid flow sensors, such as MEMS based bidirectional thermal fluid flow sensors, as a measurement principle. It was further an object of the present invention to provide a portable spirometer with a MEMS based thermal fluid flow sensor, which is optimized with regard to the flow properties inside the device in order to enable accurate and reproducible, or precise, spirometric flow measurements, preferably even by unsupervised patients.

### SUMMARY OF THE INVENTION

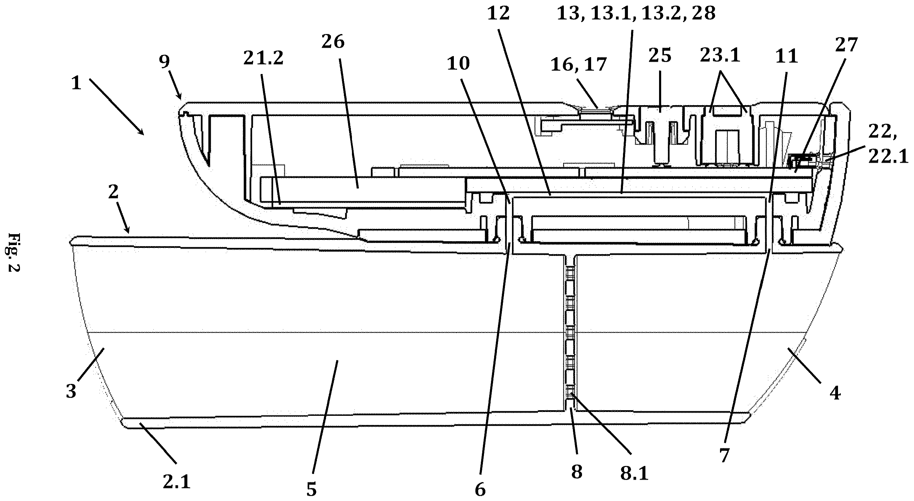

In a first aspect, the invention provides a portable, handheld electronic spirometer according to claim 1 for the evaluation of lung function parameters. One exemplary embodiment of this spirometer is depicted e.g. in figure 2.

In a second aspect, the invention provides a method for measuring a lung function associated health parameter of a human subject (for instance, a health parameter selected from a forced vital capacity (FVC), a forced expiratory volume (FEV), a peak expiratory flow (PEF), a forced expiratory flow (FEF), a maximum voluntary ventilation (MW), a mean expiratory flow (MEF), a slow vital capacity (SVC), a maximum speed of expiration, a forced inspiratory volume (FIV), a forced inspiratory vital capacity (FIVC), a peak inspiratory flow (PIF), or any combination of these), the method comprising a step of the human subject performing a breathing manoeuvre through the portable, handheld electronic spirometer as described above.

In a third aspect, the invention provides a system, or kit, comprising:
- the portable, handheld electronic spirometer (1) according to the first aspect of the invention, and
- a first air quality measurement device comprising communication means adapted for data exchange with the portable, handheld electronic spirometer (1) and/or with a separate computing unit, and equipped with one or more air quality sensors, prefereably air quality sensors selected from the group consisting of humidity sensors, temperature sensors, atmospheric pressure sensors, MOS-type gas sensors (metal-oxide-semiconductor), airborne-particles sensors, pollen sensors, ozone (O₃) sensors, nitrogen dioxide (NOz) sensors, sulfur dioxide (SOz) sensors and carbon monoxide (CO) sensors, or other type of gas sensors, for determining determine the air quality at the location of the first air quality measurement device, and optionally
- a separate computing unit adapted to collect and analyse at least the data obtained from the spirometer (1) according to the first aspect of the invention and from the first air quality measurement device.

Using said system, or kit, the method according to the second aspect of the invention may be complemented with additional data such as data related to the air quality (pollutants, ozone, pollen, etc.) and/or geolocation data, thereby allowing to compare and/or correlate the lung function associated health parameter of the human subject (such as FVC, FEV, PEF, FIV, FIVC, PIF, etc., as described above) with these additional data.

In other words, a fourth aspect of the invention provides a method wherein one or more lung function associated health parameters of a human subject (for instance, a health parameter selected as described for the second aspect of the invention), are measured by the human subject performing a breathing manoeuvre through the portable, handheld electronic spirometer according to the first aspect of the invention; and wherein the one or more lungfunction associated health parameters are compared and/or correlated with air quality data, and optionally geolocalisation data, derived from the system, or kit, according to the third aspect of the invention.

Further objects, aspects, useful embodiments, applications, beneficial effects and advantages of the invention will become apparent on the basis of the detailed description, the examples and claims below.

### OVERVIEW OF REFERENCE NUMBERS

| | | | |
|---|---|---|---|
| 1 | Spirometer | 16 | Heart rate sensor |
| 2 | Tubular mouthpiece | 17 | Blood oxygen saturation sensor |
| 2.1 | Front end of mouthpiece | 18 | Environmental temperature sensor |
| 3 | Proximal opening | 19 | Atmospheric pressure sensor |
| 4 | Distal opening | 20 | Moisture sensor |
| 5 | Main fluid channel | 21 | Radio communication means |
| 6 | First lateral opening | 21.1 | Bluetooth connectivity |
| 7 | Second lateral opening | 21.2 | NFC means |
| 8 | Perforated disk | 21.3 | WLAN means |
| 8.1 | Perforations | 22 | Cable communication means |
| 9 | Main body | 22.1 | USB communication means |
| 10 | First fluid opening | 23 | Optical signalling means |
| 11 | Second fluid opening | 23.1 | Signalling LEDs |
| 12 | Bypass fluid channel | 24 | Acoustical signalling means |
| 13 | MEMS-based thermal fluid flow sensor | 25 | ON/OFF-button |
| 13.1 | Bidirectional flow sensor | 26 | Battery |
| 13.2 | Monolithic CMOS flow sensor | 27 | Main board |
| 14 | Microcontroller | 28 | Breath temperature sensor |
| 15 | Acceleration sensor | 29 | Gyroscope |
| 15.1 | 3-axis sensor | | |

### DESCRIPTION OF THE FIGURES

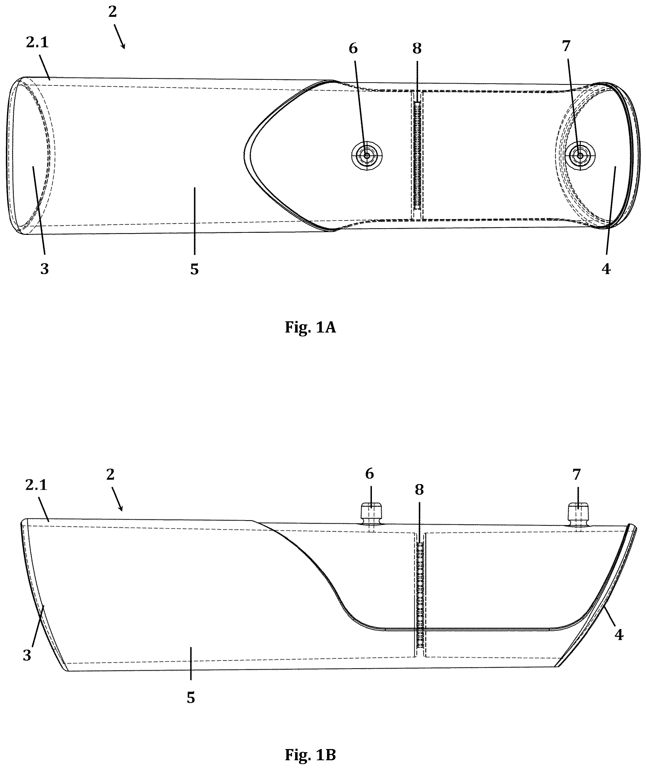

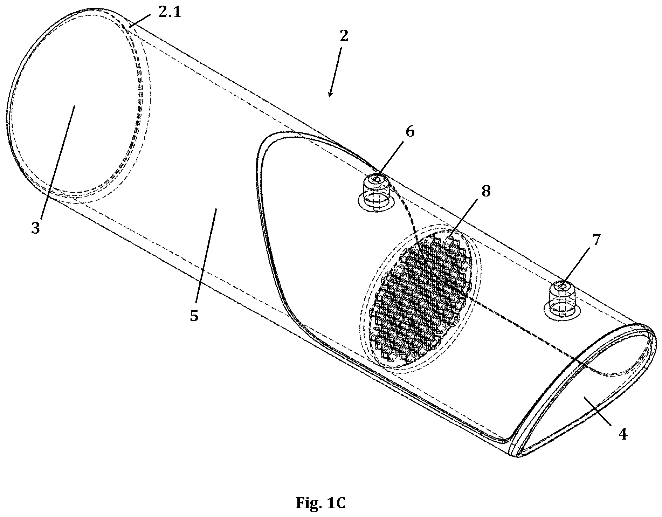

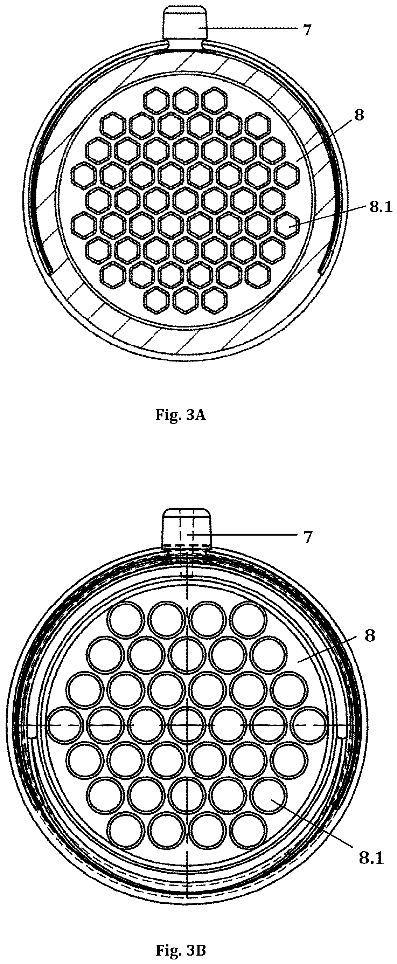

Figures 1A-C show one embodiment of the tubular mouthpiece (2) of the spirometer (1) in top view (A), side view (B) and in perspective view (C). The mouthpiece (2) comprises a proximal (3) and a distal opening (4) with a main fluid channel (5) extending therebetween, a first and a second lateral opening (6 and 7) as well as a flow restrictor in the form of a perforated disk (8) positioned in a cross-sectional orientation in the main fluid channel (5) perpendicular to the channel's longitudinal axis and between the two lateral openings (6 and 7). In the depicted embodiment, the perforated disk (8) is provided with 55 regular hexagonal perforations, as can be seen in more detail in figure 3A.
Figure 2 shows a perspective crosssection of one embodiment of the spirometer (1). On top of the tubular mouthpiece (2) with the main fluid channel (5), the perforated disk (8), and the first and second lateral opening (6 and 7) sits a detachable main body (9) with a first and a second fluid opening (10 and 11) and a bypass fluid channel (12) extending therebetween. A MEMS-based thermal fluid flow sensor (13, 13.1, 13.2), which also acts as a breath temperature sensor (28), is positioned at the top side, or upper side, of the bypass fluid channel (12). In the depicted version, the first and the second fluid opening (10 and 11) are connected to the first and second lateral opening (6 and 7) of the tubular mouthpiece (2).
Figures 3Aand 3B show crosssections of four embodiments of the spirometer (1) at the position of the perforated disk (8) as employed in specific embodiments of the spirometer (1), with either regular hexagonal perforations (8.1; Fig. 3A, here 55), or circular, or substantially circular, perforations (8.1; Fig. 3B, here 37).

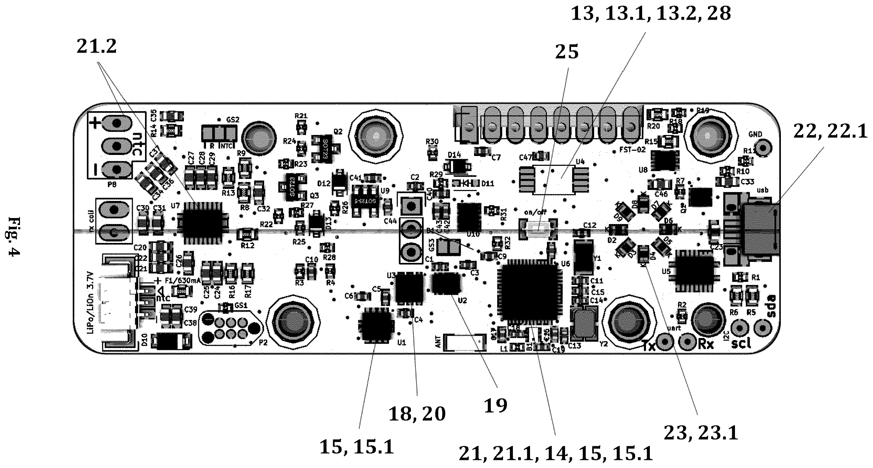

Figure 4 shows the main board (27) of one embodiment of the spirometer (1) in top view as well as the positions of the sensors (13, 13.1., 13.2, 15, 15.1, 18, 19, 20, 28), the micro-controller (14), the radio communication means (21, 21.1), the NFC means (21.2), the cable communication means (22, 22.1), and the optical signalling means (23, 23.1).

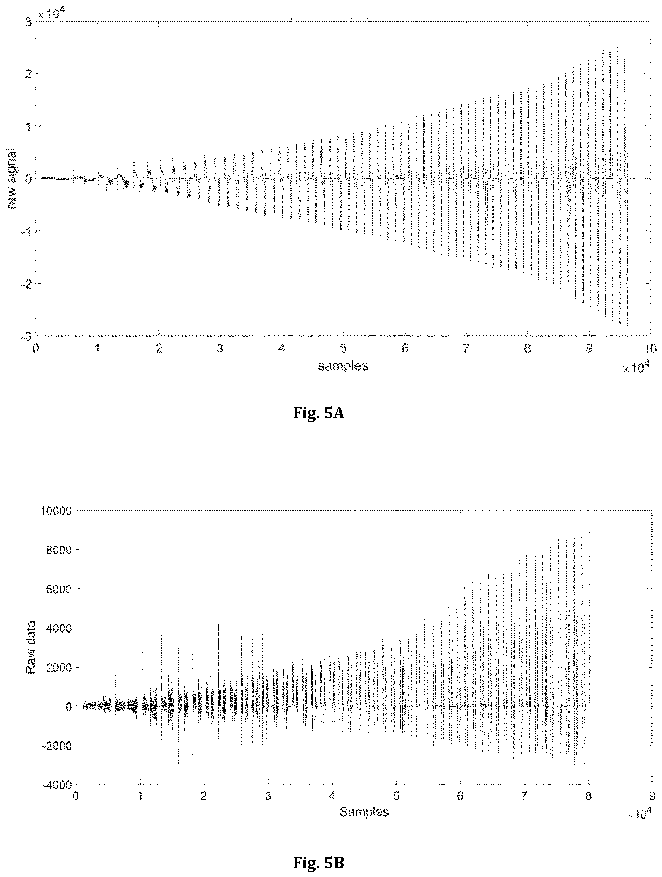

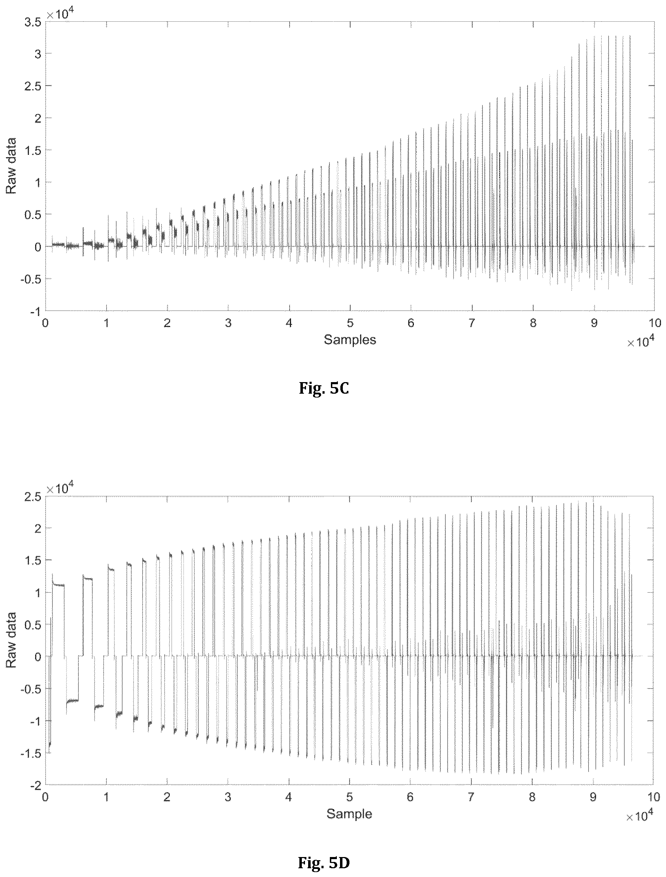

Figures 5A, B, C and D show the steady-flow waveforms measured as a standard procedure of calibration for four portable spirometers: one spirometer according to the invention (Fig. 5A), and 3 other portable spirometers for comparison which are not falling within the scope of the present invention (Fig. 5B-5D). A total of 64 waveforms starting with 0.15 L/s (left side of x-axis) and increasing gradually to 18 L/s (right side of x-axis) were measured and the resulting raw signals of the MEMS-based thermal fluid flow sensor recorded (y-axis). The four spirometers employ the same main body (9) (and hence, the same bypass fluid channel and the same MEMS-based thermal fluid flow sensor), as well as a tubular mouthpiece (2) of the same, or very similar, outer dimension such as to fit to one and the same main body (9). The four portable spirometers differ in the flow restrictors employed within the tubular mouthpiece. Fig. 5A: shows the steady-flow waveform of a spirometer (1) according to the invention comprising a tubular mouthpiece (2) with a flow restrictor in the form of a perforated disk (8); while Fig. 5B and 5C show the steady-flow waveform of a spirometer (1) comprising tubular mouthpieces (2) with flow restrictors in the form of two differently venturi elements. Fig. 5D shows the steady-flow waveform of a spirometer (1) comprising a tubular mouthpiece (2) with a movable 'flap-type' flow restrictor. (For further input, see Example 1 and Comprative Examples 1-3 below)

### DEFINITIONS

The following terms or expressions as used herein should normally be interpreted as outlined in this section, unless defined otherwise by the description or unless the specific context indicates or requires otherwise:
All technical terms as used herein shall be understood to have the same meaning as is commonly understood by a person skilled in the relevant technical field.

The words 'comprise', 'comprises' and 'comprising' and similar expressions are to be construed in an open and inclusive sense, as `including, but not limited to' in this description and in the claims.

The singular forms 'a', 'an' and 'the' should be understood as to include plural referents. In other words, all references to singular characteristics or limitations of the present disclosure shall include the corresponding plural characteristic or limitation, and vice versa. The terms 'a' 'an' and 'the' hence have the same meaning as 'at least one' or as 'one or more'. For example, reference to 'an ingredient' includes mixtures of ingredients, and the like.

The expressions, 'one embodiment', 'an embodiment', 'a specific embodiment' and the like mean that a particular feature, property or characteristic, or a particular group or combination of features, properties or characteristics, as referred to in combination with the respective expression, is present in at least one of the embodiments of the invention. These expressions, occurring in various places throughout this description, do not necessarily refer to the same embodiment. Moreover, the particular features, properties or characteristics may be combined in any suitable manner in one or more embodiments.

All percentages, parts and/or ratios in the context of numbers should be understood as relative to the total number of the respective items, unless otherwise specified, or indicated or required by the context. Furthermore, all percentages parts and/or ratios are intended to be by weight of the total weight; e.g. `%' should be read as 'wt.-%', unless otherwise specified, or indicated or required by the context.

'Essentially', 'about', 'approximately' (approx.), 'circa' (ca.) and the like in connection with an attribute or value include the exact attribute or the precise value, as well as any attribute or value typically considered to fall within a normal range or variability accepted in the technical field concerned.

'Front' as well as all similar terms designating a position, orientation or direction, such as 'left', 'right', 'rear' 'back', 'top', 'bottom', 'up', 'down' and the like, should be understood with reference to the orientation of the spirometer or its components under normal operational conditions. 'Lateral', or 'laterally', means away from the middle, centre, or centre axis of a device or device component.

The terms 'sensor' and 'transducer' are used synonymously herein, unless where specified otherwise, and refer to means which are capable of measuring a parameter (for instance, a force, a temperature or a sound) and transmitting a related signal to a data analysis unit, e.g. an electric signal which can be received, read, stored and analysed by a computer or a similar data analysis unit. In that regard, it should be understood that wordings such as 'a signal obtained from a sensor ...' strictly speaking refers to the signal as transmitted to the computer, and thus not necessarily to the actual measured parameter, or measurand, such as a force which triggered the respective signal.

The term 'spirometry' or 'full spirometry' refers to the entirety of measurements related to the breathing capacities, or pulmonary function, of the lungs of a breathing subject, both during inhalation or exhalation, as well as during forced or quiet breathing manoeuvres. These measurements are done both qualitatively as well as quantitatively. The term 'spirometer' as used herein thus refers to devices which are capable to perform these measurements. Examples of the most common parameters measured in (full) spirometry are vital capacity (VC), forced vital capacity (FVC), forced inspiratory vital capacity (FIVC), forced expiratory volume (FEV) at timed intervals in seconds (e.g. FEV₁ = FEV in 1 second), forced expiratory flow (FEF), peak expiratory flow (PEF; also called peak flow), forced expiratory time (FET) and maximal voluntary ventilation (MW; also called maximum breathing capacity). In other words, spirometry includes, or encompasses, peak flow measurements; therefore, it is understood that the spirometer according to the present invention may also be employed as a peak flow meter, while not being limited to this functionality alone. The vice-versa case is not necessarily valid; i.e. a peak flow meter is not a spirometer if limited to the functionality of measuring peak flows. Likewise, while the `spirometers' in the sense of the present invention could in theory be employed for so-called incentive spirometry (a technique in which a subject is instructed to repeatedly inhale slowly and optionally hold its breath in order to inflate the lungs and keep the small airways open, e.g. after lung surgery or in bed-ridden patients), not every incentive spirometer can necessarily perform the above described qualitative and quantitative measurements of lung function parameters, and hence does not necessarily qualify as a 'spirometer' in the sense of the present invention, despite the similarity in names.

The term 'portable' as used herein refers to products, in particular spirometers, whose size and weight renders them suitable to be carried comfortably and for extended periods of time (such as the whole day and/or on a daily basis) by human users of said product without additional help; for instance, by simply holding it in one hand or by placing it in the pockets of trousers or coats or in a handbag. Hence, terms such as pocket-sized and/or handheld are understood to be synonymous. Typically, products with a size of about 200 x 60 x 50 mm or smaller and an overall weight of about 250 g or lighter, preferably about 150 g or even about 100 g or lighter, are considered portable. The term 'portable' further means that, during use and/or "on the go", the device is fully operable without an attached cable power source and/or without the need to be connected to a stationary workstation (such as a dedicated decking station, personal computer, or the like); for instance, the portable spirometer of the invention does not need to be plugged into a power socket for the user to be able to perform full spirometry measurements.

So-called table-top devices, in particular table-top spirometers, as commonly employed in clinical settings, are not considered 'portable' in the sense of the present invention. While theoretically some of these table-top devices could still be lifted and carried aound by a human user without additional help, too, it would typically not be considered comfortable for longer times (e.g. a whole day), and/or would require the use of a dedicated casing (e.g. a suitcase) and/or the use of both hands.

The expression 'positioned at' - as used herein e.g. in 'the MEMS-based thermal fluid flow sensor (13, 13.1, 13.2) is positioned at the bypass fluid channel (12)' - means that the sensor is not only in the vicinity to the said bypass fluid channel (12), but in fluid connection with the fluid flow passing through the bypass fluid channel (12). For this purpose, the bypass fluid channel (12) exhibits an opening in its lateral walls into which the the MEMS-based thermal fluid flow sensor (13, 13.1, 13.2) is placed. The MEMS-based thermal fluid flow sensor (13, 13.1, 13.2) may, for instance, be positioned flush with the lateral walls of the bypass fluid channel (12), or alternatively may protrude further through the opening in the lateral walls of the bypass fluid channel (12) and into the lumen of the bypass fluid channel (12).

Any reference signs in the claims should not be construed as a linlitation to the embodiments represented in any of the drawings.

### DETAILED DESCRIPTION OF THE INVENTION

In a first aspect, the invention provides a portable, handheld electronic spirometer (1) for the evaluation of lung function parameters comprising (a) a tubular mouthpiece (2) with a proximal opening (3) for insertion into the mouth of a user, a distal opening (4), a main fluid channel (5) extending between the proximal opening (3) and the distal opening (4) and exhibiting a cross sectional area (Aₘ) in the range of from about 200 mm² to about 1400 mm², a first lateral opening (6), a second lateral opening (7) positioned at a longitudinal distance to the first, and a flow restrictor in the form of a perforated disk (8) positioned in a cross-sectional orientation in the main fluid channel (5) between the first and the second lateral opening (6 and 7); and (b) a main body (9) with a first fluid opening (10) connectible with the first lateral opening (6) of the mouthpiece (2), a second fluid opening (11) connectible with the second lateral opening (7) of the mouthpiece (2), a bypass fluid channel (12) extending between the first and the second fluid opening (10 and 11) and exhibiting a cross-sectional area (A_{b}) in the range of from about 1 mm² to about 16 mm², a MEMS-based thermal fluid flow sensor (13) which is positioned at the bypass fluid channel (12) for generating a signal in response to the fluid flow in the bypass fluid channel (12), and a microcontroller (14) connected with the fluid flow sensor (13) for calculating the fluid flow from the signal generated by the flow sensor (13), and a communication means for the exchange of data related to the fluid flow generated by the micro-controller (14) of the spirometer (1); wherein
i) the cross-section of the bypass fluid channel (12) with respect to the cross-section of the main fluid channel (5), and
ii) the perforated disk (8) are adapted, or configured, in a such way that the flow resistance of the spirometer (1) does not exceed 0.15 kPa/(L/s). One exemplary embodiment of this spirometer (1) is depicted in figure 2 for instance.

Optionally, the tubular mouthpiece (2) and the main body (9) may be detachable from one another. Further optionally, the connection between the first fluid opening (10) of the main body (9) with the first lateral opening (6) of the mouthpiece (2) and/or between the second fluid opening (11) of the main body (9) with the second lateral opening (7) of the mouthpiece (2) may be achieved by a snap-fit mechanism. Preferably, the mouthpiece (2) is designed to fit the main body (9) in only one way, or direction; preventing misplacement and/or incorrect assembly of the two parts.

According to the present invention, the flow resistance of the spirometer (1) should not exceed the maximum value of 0.15 kPa/(L/s) at any fluid flow rate within the range of > 0 SLM to about 840 SLM (or SLPM; standard liter per minute; or 0-14 L/s) since this is a requirement, for instance for FVC- and FEV₁-measurements described by the "Standardisation of spirometry" (as pulished e.g. in Eur Respir J 2005; 26: 319-338). This requirement means that the flow resistance shall not exceed 0.15 kPa/(L/s) over the whole fluid flow rate range of > 0 SLM to about 840 SLM, not just at a single flow rate value selected therefrom; or in other words, if the flow resistance of a tested spirometer is below 0.15 kPa/(L/s) at a lower flow rate of e.g. 1 L/s but exceeds 0.15 kPa/(L/s) at a higher flow rate of e.g. 800 L/s, the requirement is not complied with.

However, during spirometric measurements (i.e. as used herein, measurements for the evaluation of lung function parameters), flow rates up to about 900 SLM, or up to about 960 SLM, sometimes even up to about 1000 SLM may be achieved when the subject, or patient, breathes forcefully through the spirometer (1). Therefore, in one of the preferred embodiments, the cross-section of the bypass fluid channel (12) with respect to the cross-section of the main fluid channel (5), and the perforated disk (8) are adapted, or configured, in a such way that the flow resistance of the spirometer (1) does not exceed 0.15 kPa/(L/s) even at fluid flow rates within these higher ranges of from > about 840 SLM to about 900 SLM, or preferably within the range of from > about 840 SLM up to about 960 SLM, or more preferably even within the range of from > about 840 SLM up to about 1000 SLM.

With regard to the fluid flow rates described herein, it should further be understood that the spirometer (1) according to the present invention is capable of measuring both exhalative flows and inhalative flows. Hence, unless where explicitly indicated otherwise, any flow rate value provided herein, should be understood as its absolute value and as referring to both exhalative and inhalative flow rates; such as +840 SLM and -840 SLM; or within the range of > 0 SLM to about +840 SLM and within the range of < 0 SLM to about -840 SLM for exhalation and inhalation flows, respectively.

In one embodiment of the invention, i) the cross-section (A_{b}) of the bypass fluid channel (12) with respect to the cross-section (Aₘ) of the main fluid channel (5), and
ii) the perforated disk (8) are adapted, or configured, in a such way as to cause a fluid flow in the bypass fluid channel (12) which is from about 1 : 2.5 to about 1 : 200, or from about 1 : 4.5 to about 1 : 200, or from about 1 : 10 to about 1 : 200, of the fluid flow in the main fluid channel (5).

Alternatively, or in addition thereto, i) the cross-section (A_{b}) of the bypass fluid channel (12) with respect to the cross-section (Aₘ) of the main fluid channel (5), and
ii) the perforated disk (8) are adapted, or configured, in a such way as to cause a fluid flow in the bypass fluid channel (12) which ranges from about 0.3 SLM to about 350 SLM, or from about 0.3 SLM to about 200 SLM, or from about 0.3 SLM to about 90 SLM.

According to the invention, the spirometer (1) exhibits a flow resistance, or impedance, in the range from about 0.01 to about 0.14 kPa/(L/s) at any fluid flow rate within the range of > 0 SLM to about 840 SLM (or SLPM; standard liter per minute), or preferably from about 0.01 to about 0.12 kPa/(L/s), or more preferably from about 0.01 to about 0.1 kPa/(L/s). In other words, the flow resistance of the spirometer (1) should not exceed the maximum value of 0.14 kPa/(L/s), or preferably 0.12 kPa/(L/s), or more preferably 0.10 kPa/(L/s) at any fluid flow rate within the range of > 0 SLM to about 840 SLM. In a further embodiment, the spirometer (1) exhibits the above described flow resistance values in the range from about 0.01-0.14 kPa/(L/s), or preferably from about 0.01-0.12 kPa/(L/s), or more preferably from about 0.01-0.1 kPa/(L/s) even at higher fluid flow rates; for instance, at fluid flow rates within the ranges of from > about 840 SLM to about 900 SLM, or preferably within the range of from > about 840 SLM up to about 960 SLM, or more preferably even within the range of from > about 840 SLM up to about 1000 SLM.

One advantage of the spirometer (1) according to the invention is that at a size of about 115 x 55 x 45 mm and a weight below 100 g, the device is both light weight and small, pocket-sized, handheld and thus easily portable by a user (e.g. in a coat pocket, trousers pocket or a handbag), while at the same time allowing for full spirometry as defined by the spirometry standards of the American Thoracic Society (ATS), the European Respiratory Society (see e.g. Eur. Respir. J. 1997; 10: Suppl. 24, 2s-8s; or "Standardisation of spirometry"; Eur. Respir. J. 2005; 26: 319-338) or ISO 26782:2009 (specifying the requirements for spirometers intended for the assessment of pulmonary function in humans weighing more than 10 kg) at very high precision; including measurements during both inhalation and exhalation and providing all functions of spirometers as used in hospital settings. The device is further capable to fulfil the peak expiratory flow statement by the ERS (see e.g. "Peak expiratory flow: conclusions and recommendations of a Working Party of the European Respiratory Society").

The basic functionality of the spirometer (1) includes the measurement of exhalatory and inspiratory fluid flow rates, time and volume of exhalation and inspiration, as well as calculation of all spirometric parameters of interest, including the most common: FVC, FEV₁, PEF anf FEV₁% but also parameters such as FEV₆, SVC or MW, in order to assess the respiratory function of a user (e.g. a patient suffering from a respiratory disease, or an athlete).

Additionally, the spirometer (1) will continuously monitor the local environmental parameters, such as temperature, pressure and ambient air humidity, as will be detailed further below. This may, for instance, be achieved by monitor the local environmental parameters at a predefined monitoring frequency or monitoring intervall (e.g. 10 seconds for every one hour, every half hour or every quarter hour, or the like). Like this, the user of the spirometer (1) not only measures and receives the spirometric data with regards to his/her lungfunction but can also match specific data points to e.g. the environmental parameters at, or around, the spirometric measurement time point In addition, these environmental sensors may be used e.g. for so-called BTPS-conversion of FVC measurements (BTPS: body temperature pressure saturated); i.e. the vital capacity at maximally forced expiratory effort, expressed in litres at body temperature and ambient pressure saturated with water vapour, as required by the ATS/ERS-standards of spirometry in order to allow for comparability across different temperatures, pressures and humidity conditions; i.e. a standardisation of environmental conditions (see e.g. "Standardisation of spirometry"; Eur Respir J 2005; 26: 319-338). The portable spirometers of the prior art do not have these sensors build in; hence, the user is required to enter these data manually, which makes the device less versatile and increases the risk of erroneous measurements, or data interpretation, in particular with lay-people.

A further advantage is that the spirometer (1) may be used by lay people, i.e. without medical or similarly trained staff as currently required for most spirometry tests in doctor's offices and/or hospital settings; thus providing users with an 'in-home' spirometer which they can use by themselves. In that regard, it should be understood that in the context of this invention, users are not necessarily patients afflicted with respiratory diseases. The parameters used to examine the respiratory tract are also helpful e.g. for athletes training regularly, enabling them to monitor their training progress and track their performance; or for smokers wanting to evaluate the benefits of their smoking cessation.

Advantageously, the spirometer (1) may be connected to a user's personal computer and/or smartphone using the spirometers's communication means for the exchange of data related to the fluid flow generated by the micro-controller (14). Preferably, said connection is supported by a dedicated proprietary spirometer application (`app') with proprietary and predictive algorithms; or as an 'add-on' integrated in existing healthcare apps available for iOS or Android smartphones.

Furthermore, the spirometer (1) of the invention is fully electronic and does not comprise any moving parts, such as rotating turbines or oscillating cantilevers as they are common for measuring the fluid flow in prior art spirometers, thus obviating the need for regular, frequent external calibrations. Further, it turns on quickly with less than 7 seconds between switching the spirometer (1) on and the device being ready for use. This is not only energy-efficient and thus saving battery life, but also renders the device suited to be used "on the go" by medical staff such as doctors; for instance, during ward rounds, home visits, etc.

MEMS-based thermal fluid flow sensors (13) provide a high sensitivity for fluid flow measurements, yet at the same time suffer from an inherent detrimental susceptibility to vibration; i.e. any flow measurement attempts are per se affected by non-flow-related vibrations as they occur e.g. when the user moves the spirometer during use. This may be one of the reasons, why - acording to the current knowledge of the inventors - no portable, handheld yet operable, fully functional spirometer comprising a MEMS-based thermal fluid flow sensor has actually been developed before. The invention is based on the unexpected discovery that a MEMS-based thermal fluid flow sensor (13) can be incorporated in a portable electronic, handheld spirometer in such a way that accurate and reproducible, or precise, (full) spirometric flow measurements are enabled, and unlike many other devices further allows both inspiratory and expiratory lung function assessments. This is achieved by positioning the MEMS-based thermal fluid flow sensor (13) at a bypass fluid channel (12) exhibiting a cross-sectional area (A_{b}) in the range of from about 1 mm² to about 16 mm², and by providing a flow restrictor in the form of a perforated disk (8) which is positioned in a cross-sectional orientation in a main fluid channel (5) exhibiting a cross-sectional area (Aₘ) in the range of from about 200 mm² to about 1400 mm², in order to redirect specific fractions of the air flow in the main fluid channel (5) to the bypass fluid channel (12). When selecting the claimed surface area ranges for the main and bypass fluid channels (5, 12),and when incorporating the MEMS-based thermal fluid flow sensor (13) in the spirometer (1) in this way, the sensor (13) provides a higher precision, reproducibility and sensitivity than the different flow sensors typically used in prior art portable spirometers, such as fan-based transducers (turbines). In addition, by placing the MEMS-based thermal fluid flow sensor (13) in the bypass fluid channel (12) comprised in the main body (9) of the spirometer (1), it is also protected from direct exposure to saliva and/or bioparticles that could damage it or affect the accuracy and/or precision of measurements.

The precision, reproducibility and sensitivity is mainly achieved by carefully adapting, or configuring: i) the cross-section (A_{b}) of the bypass fluid channel (12) with respect to the cross-section (Aₘ) of the main fluid channel (5) and ii) the perforated disk (8); further input on suitable selections will be described in more detail below. Nonetheless, the precision, reproducibility and sensitivity may be increased further by using an acceleration sensor (15) which is different from the MEMS-based thermal fluid flow sensor (13) and which is not connected to the main fluid channel (5) or the bypass fluid channel (12) in addition to said MEMS-based thermal fluid flow sensor (13). This acceleration sensor (15), preferably an acceleration sensor (15) which is incorporated within the portable, handheld electronic spirometer (1), in particular within the main body (9) the portable, handheld electronic spirometer (1), allows for the correction of the calculated fluid flow as will be detailed further below. The acceleration sensor (15) further allows for alerting the user if movement is detected during measurements and, if needed, to instruct the user to correct his/her position and repeat the measurement, and/or to disregard incorrectly performed manoeuvres in longterm analysis (e.g. manoeuvres with substantial head movement); thereby improving the quality of the single manoeuvre as well as the longterm analysis of lungfunction parameters. In final consequence, the acceleration sensor (15) also allows for a clinically relevant improvement of the sensitivity, accuracy, and reproducibility, or precision, of the spirometric flow measurements of the spirometer (1).

In one embodiment, the mean accuracy of the spirometer (1) fulfills ATS/ERS criteria; i.e. the parameters determined with the spirometer (1) of the invention differ not more than the allowed values from the reference flowcurves (see accuracy tests for spirometers in "Standardisation of spirometry"; Eur Respir J 2005; 26: 319-338, on page 333; or ISO 26782:2009 which specifies requirements for spirometers intended for the assessment of pulmonary function in humans weighing >10 kg). Even at low flow rates below 0.3 L/sec, the accuracy is maximally ± 3%. The repeatability, or in other words the reproducibility or precision, is ± 0.5 %.

In fact, the use of a MEMS-based thermal fluid flow sensor (13) in the spirometer (1) of the invention as claimed, and preferably a spirometer (1) with an incorporated acceleration sensor (15) which is not connected to the main fluid channel (5) or the bypass fluid channel (12) in addition to said MEMS-based thermal fluid flow sensor (13), renders the device sensitive enough to even measure the minute movements of air moved in or out of the trachea by the heart beat, enabling new medical uses which were not available before with prior art spirometers. To the best of the inventors' knowledge, no other portable spirometer of the prior art is this sensitive.

A yet further advantage of the inventive spirometer (1), in particular, for embodiments where the mouthpiece (2) and the main body (9) are detachable from one another, is that because the MEMS-based thermal fluid flow sensor (13) is positioned at the bypass fluid channel (12) comprised in the main body (9), the mouthpiece (2) may be detached easily and safely from the main body (9) without the risk of potentially damaging said flow sensor (13), or otherwise affecting the accuracy and/or precision of its measurements. This overcomes the limitations of prior art devices in which only the accurate (re)placement of a detachable mouthpiece into a main body ensured proper functioning as well as accuracy and precision of the flow sensor, e.g. a pressure sensor. The spirometer (1) of the invention comprises a mouthpiece (2) designed to fit the main body (9) in only one way, or direction; preventing misplacement and/or incorrect assembly of the two parts, as described above.

The main fluid channel (5) of the tubular mouthpiece (2) is typically shaped as a hollow circular, or substantially circular, cylinder or as an elliptical cylinder, partially in order to resemble the shape of the opened mouth of a user upon inhaling or exhaling through the main fluid channel. Optionally, the cylinder may be slightly tapered towards the distal opening (4); for instance, gradually narrowing from an outer diameter of about 31 mm at the proximal opening (3) to an outer diameter of about 29 mm at the distal opening (4) over a length of about 110 to 120 mm.

In general, the diameter of the main fluid channel (5) at the proximal opening (3) should be chosen such as to comfortably fit the mouth of the intended user and allow him/her to effectively seal the mouthpiece (2) with the lips. For instance, an outer diameter at the proximal opening (3) of about 30 mm for adult users would be suitable and smaller diameters for infants or kids. Optionally, a small groove, or ridge, may be provided for the user's teeth in order to improve the seal between lips and mouthpiece (2).

At the same time, the inner diameter of the main fluid channel (5) at the distal opening (4) should be chosen large enough such as to not cause too much flow resistance; for instance, said inner diameter should preferably not be smaller than the users' trachea.

In one embodiment of the above-described spirometer (1), the cross-sectional area (Aₘ) of the main fluid channel (5) ranges from about 250 mm² to about 1300 mm², or from about 350 mm² to about 1100 mm², or from about 450 mm² to about 800 mm², or from about 530 mm² to about 760 mm². In one of the preferred embodiments, the cross-sectional area (Aₘ) of the main fluid channel (5) is in the range of from about 550 mm² to about 630 mm², such as 587 mm². In cases, where the tubular mouthpiece (2) exhibits a tapered shape (typically, gradually narrowing from a larger diameter at the proximal opening at the up-stream end to the distal opening at the down-stream end), at least the cross-sectional area (Aₘ) of the main fluid channel (5) at the position of the perforated disk (8) is falling into the above-mentioned ranges; for instance, in a particular embodiment, the cross-sectional area (Aₘ) of the main fluid channel (5) at the position of the perforated disk (8) is in the range of from about 550 mm² to about 630 mm², such as 587 mm².

In the embodiment as depicted in figure 1A-C, the front end (2.1) of the tubular mouthpiece (2), i.e. the end comprising the proximal opening (3) is configured as an integral part of the tubular mouthpiece (2). Alternatively, this front end (2.1) may be configured as a detachable part of the tubular mouthpiece (2), allowing to remove this front end (2.1) portion of the mouthpiece (2) in order to either clean it, or discard and replace it, after contact with a user's lips and/or tongue. A detachable front end (2.1) facilitates cleaning and enables the use of disposable parts in multi-patient settings (where applicable).

Alternatively, or in addition to the above-described selected the cross-sectional area (Aₘ) of the main fluid channel (5), the cross-sectional area (A_{b}) of the bypass fluid channel (12) may be selected within the range of from about 1 mm² to about 12 mm², or from about 1 mm² to about 11 mm², or from about 1 mm² to about 10 mm², or from about 1 mm² to about 9 mm², or from about 1 mm² to about 8 mm², or from about 1 mm² to about 7 mm², or from about 1 mm² to about 6 mm², or from about 1 mm² to about 5 mm², or from about 1 mm² to about 4 mm². For instance, in one embodiment, the cross-sectional area (Aₘ) of the main fluid channel (5) ranges from about 250 mm² to about 1300 mm²; and/or the cross-sectional area (A_{b}) of the bypass fluid channel (12) ranges from about 1 mm² to about 9 mm². In one of the preferred emebodiments, the cross-sectional area (A_{b}) of the bypass fluid channel (12) is selected from about 1 mm², or about 1.1 mm², or about 1.2 mm², or about 1.3 mm², or about 1.4 mm², or about 1.5 mm², or about 1.6 mm², or about 1.7 mm², or about 1.8 mm², or about 1.9 mm², or about 2 mm², or about 2.1 mm², or about 2.2 mm², or about 2.3 mm², or about 2.4 mm², or about 2.5 mm², or about 3.2 mm², or about 4 mm².

In one embodiment, the ratio of the cross-sectional area (Aₘ) of the main fluid channel (5) to the cross-sectional area (A_{b}) of the bypass fluid channel (12) may be selected within the range of from about 12 to about 1400, or from about 15 to about 1300, or from about 21 to about 1100, or from about 28 to about 800, or from about 33 to about 760. In a specific embodiment, the ratio of the cross-sectional area (Aₘ) of the main fluid channel (5) to the cross-sectional area (A_{b}) of the bypass fluid channel (12) ranges from about 22 to about 1400, or from about 27 to about 1300, or from about 38 to about 1100, or from about 50 to about 800, or from about 58 to about 760. In a further specific embodiment, the ratio of the cross-sectional area (Aₘ) of the main fluid channel (5) to the cross-sectional area (A_{b}) of the bypass fluid channel (12) ranges from about 50 to about 1400, or from about 62 to about 1300, or from about 87 to about 1100, or from about 112 to about 800, or from about 132 to about 760.

According to the invention, the ratio of the cross-sectional area (Aₘ) of the main fluid channel (5) to the cross-sectional area (A_{b}) of the bypass fluid channel (12) ranges from about 100 to about 800, or more specifically from about 115 to about 700, or from about 130 to about 600.

In one of the preferred embodiments, the cross-sectional area (Aₘ) of the main fluid channel (5) is selected within the range of from about 530 mm² to about 760 mm², or from about 550 mm² to about 630 mm² (e.g. about 587 mm²); and
the cross-sectional area (A_{b}) of the bypass fluid channel (12) ranges from about 1 mm² to about 4 mm². For instance, the cross-sectional area (Aₘ) of the main fluid channel (5) may be about 587 mm² and the the cross-sectional area (A_{b}) of the bypass fluid channel (12) may be selected from about 1 mm², about 1.1 mm², about 1.2 mm², about 1.3 mm², about 1.4 mm², about 1.5 mm², about 1.6 mm², about 1.7 mm², about 1.8 mm², about 1.9 mm², about 2 mm², about 2.1 mm², about 2.2 mm², about 2.3 mm², about 2.4 mm², about 2.5 mm², about 3.2 mm², or about 4 mm².

In a specific embodiment, the cross-sectional area (Aₘ) of the main fluid channel (5) ranges from about 530 mm² to about 760 mm²,or from about 550 mm² to about 630 mm² (e.g. about 587 mm²), the cross-sectional area (A_{b}) of the bypass fluid channel (12) is about 9 mm², and the fluid flow in the bypass fluid channel (12) ranges from about 0.3 SLM to about 350 SLM. In a further specific embodiment, the cross-sectional area (Aₘ) of the main fluid channel (5) ranges from about 530 mm² to about 760 mm², or from about 550 mm² to about 630 mm² (e.g. about 587 mm²), the cross-sectional area (A_{b}) of the bypass fluid channel (12) is about 4 mm², and the fluid flow in the bypass fluid channel (12) ranges from about 0.3 SLM to about 200 SLM. In a yet further specific embodiment, the cross-sectional area (Aₘ) of the main fluid channel (5) ranges from about 530 mm² to about 760 mm², or from about 550 mm² to about 630 mm² (e.g. about 587 mm²), the cross-sectional area (A_{b}) of the bypass fluid channel (12) is about 1 mm², and the cross-sectional area (A_{b}) of the bypass fluid channel (12) is about 9 mm² and the fluid flow in the bypass fluid channel (12) ranges from about 0.3 SLM to about 90 SLM.

The bypass fluid channel's (12) cross-section may exhibit a circular, or substantially circular, half-circular, elliptic, rectangular, or substantially rectangular, or a polygonal shape, or even a `mixed shape' such as a U-shaped form with a round 'bottom' and flat 'ceiling', The choice of the shape may in parts be guided by the preparation process, as well as the preference for achieving laminar flow in the bypass fluid channel (12), or at or around the MEMS-based thermal fluid flow sensor (13) positioned within the bypass fluid channel (12), during use of the spirometer (1).

In one embodiment, the bypass fluid channel (12) has a rectangular, or substantially rectangular, cross section. In a specific embodiment, the bypass fluid channel (12) has a quadratic, or substantially quadratic, cross section; it is presumed, that this may offer a slightly steadier, more evenly distributed and more laminar fluid flow in the bypass fluid channel (12) than rectangular, or substantially rectangular, cross-sections.

In a further specific embodiment, the bypass fluid channel (12) exhibits a quadratic, or substantially quadratic, cross-section of in the range of from about 1 × 1 mm to about 4x4 mm. In a yet further specific embodiment, the bypass fluid channel (12) exhibits a quadratic, or substantially quadratic, cross-section in the range of from about 1 × 1 mm to about 3 x 3 mm, or in the range of from about 1 × 1 mm to about 2 x 2 mm. In one of the preferred embodiments, the bypass fluid channel (12) exhibits a quadratic, or substantially quadratic, cross-section selected from about 1 × 1 mm, about 1.05 x 1.05 mm, about 1.10 x 1.10 mm, about 1.14 x 1.14 mm, about 1.18 x 1.18 mm, about 1.22 x 1.22 mm, about 1.26 x 1.26 mm, about 1.30 x 1.30 mm, about 1.34 x 1.34 mm, about 1.38 x 1.38 mm, about 1.41 x 1.41 mm, about 1.45 x 1.45 mm, about 1.48 x 1.48 mm, about 1.52 x 1.52 mm, about 1.55 x 1.55 mm, about 1.58 x 1.58 mm, about 1.78 x 1.78 mm, or about 2 x 2 mm.

As mentioned, a flow restrictor in the form of a perforated disk (8) is also comprised in the spirometer (1) which - in combination with the bypass fluid channel (12) - enables accurate and reproducible, or precise, measurements of the air flow in the main fluid channel (5) by the MEMS-based thermal fluid flow sensor (13; or hereafter also shortly referred to as the flow sensor (13)). The perforated disk (8) is employed in order to direct some of the fluid flow, namely the inhaled or exhaled air stream, which passes through the main fluid channel (5) into the bypass fluid channel (12) and past the flow sensor (13). This is important because the flow sensor (13) is highly sensitive; i.e. by redirecting only a fraction of the fluid flow in the main fluid channel (5) through the bypass fluid channel (12), the flow sensor (13) is enabled to generate signals which have a high correlation with the fluid flow in the main fluid channel (5). Furthermore, the flow sensor (13) is sensitive to vibrations, or noises, which may result from a movement or acceleration of the device; therefore, shielding it from the main fluid channel (5) further helps to ensure precise and accurate fluid flow measurements.

In one embodiment, the perforated disk (8)is fixed, or immobile, or immovable within the main fluid channel (5); in other words, it is a mechanical flow restrictor (8) which exhibits sufficient stiffness to not move or deform within the main fluid channel (5) when subjected to fluid flow (unlike e.g. the movable `flap-type' flow restrictors such as depicted in Fig. 18 of EP 0552916 A1).

In one embodiment, the perforated disk (8) exhibits a diameter matching the inner diameter of the main fluid channel (5), such as to allow fluid flow only through the perforations (8.1) of the disk (8).

Optionally, the perforated disk (8) is arranged perpendicular to the main fluid channel's (5) longitudinal axis, as depicted, for instance, in Fig. 2.

A perforated disk (8) is advantageous, for instance, in comparison to a venturi section in the main fluid flow channel, in that it can be exchanged more easily to e.g. adjust flow restriction values (such as for adults, kids, infants). Optionally, the tubular mouthpiece (2) may comprise a dedicated groove into which the perforated disk (8) can be slid such as to be held there in fixed state, or immobilized, within the mouthpiece (2) during transport and/or use of the spirometer (1). The perforated disk (8) further allows to sustain a laminar air flow which is vital to avoid unpredicted turbulances in the main fluid channel (5) and the bypass fluid channel (12). Furthermore, perforated disks may be preferred in that they allow very smooth airflow with little turbulence, and a signal with only limited noise in the main fluid channel (5). In addition, the perforated disky are typically easy to prepare; for instance, using molding or 3D-printing techniques.

In one embodiment, the perforated disk (8) exhibits from about 15 to about 100 perforations (8.1). For instance, the perforated disk (8) may exhibit from about 20 to about 90 perforations, or from about 20 to about 80 perforations, or from about 25 to about 80 perforations, or from about 25 to about75 perforations; or from about 30 to about 75 perforations, or from about 30 to about 70 perforations, or from about 35 to about 65 perforations. These perforations (8.1) may be substantially circular, elliptic or polygonal in shape. In case of polygonal perforations (8.1), regular polygons, such as regular hexagons, regular octagons, or the like are preferred; i.e. polygons with equal edge lengths. Optionally, perforations (8.1) of more than one shape may be combined with each other, such as a multitude of circular perforations (8.1) with a multitude of regular hexagonal perforations (8.1), or the like. Further optionally, the perforations (8.1) may have the same size for all perforations, or exhibit sets of differently sized perforations (8.1).

According to the invention, these perforations (8.1) may exhibit a total combined area of all perforations (8.1) ranging from about 26 % to about 72 %, or more specifically from about 28 % to about 60 %, or from about 28 % to about 50 %, or from about 30 % to about 50 %, or from about 32 % to about 48 %, or from about 34 % to about 46 %, or from about 36 % to about 44 %, or from about 38 % to about 42 %, of the cross-sectional area of the main fluid channel (5) at the position of the perforated disk (8). In other words from about 26 % to about 72 %, or from about 28 % to about 60 %, or from about 28 % to about 50 %, or from about 30 % to about 50 %, or from about 32 % to about 48 %, or from about 34 % to about 46 %, or from about 36 % to about 44 %, or from about 38 % to about 42 %, of the cross-sectional area of the perforated disk (8) is open/perforated and hence lets the air or fluid flow pass (this area also being referred to herein as the `perforated area' (Aₚ)); such as from about 28 % to about 35 % (e.g. about 30 %, or about 32 %, or about 34 %) or from about 40 % to about 50 % (e.g. about 43 %, or about 45 %, or about 47 %, or about 49 %).

For all embodiments, the `perforated area' (Aₚ) is controllable both via the adjustment of the number of perforations (8.1) and/or via the adjustment of the size, or surface area, of the perforations (8.1).

In a specific embodiment, the perforated disk (8) is provided with about 35 to about 80, or about 45 to about 70, perforations, or about 50 to about 60, perforations (8.1), said perforations (8.1) exhibiting a `perforated surface area' (Aₚ) of from about 26 % to about72 % of the perforated disk's (8) total surface area. In a further specific embodiment, the perforated disk (8) exhibits a total surface area in the range of from about 550 mm² to about 630 mm², such as about 587 mm² and from about 40 to about 70 perforations (8.1) (e.g 55), said perforations (8.1) exhibiting a `perforated surface area' (Ap) of about 160 mm² to about 205 mm²
(e.g. about 175 mm²), or about from about 28 % to about 35 % (e.g. about 30 %) of the perforated disk's (8) total surface area. In a yet further specific embodiment, the perforations (8.1) are shaped as regular hexagons, as depicted exemplarily in Fig. 3A.

In another specific embodiment, the perforated disk (8) exhibits a total surface area in the range of from about 550 mm² to about 630 mm², such as about 587 mm² and from about 35 to about 50 perforations (8.1) (e.g. 37), said perforations (8.1) exhibiting a `perforated surface area' (Ap) of from about 235 mm² to about 285 mm² (e.g. about 262 mm²), or from about 40 % to about 50 % (e.g. about 45 %) of the perforated disk's (8) total surface area. In a more specific embodiment, the perforations (8.1) exhibit a circular, or substantially circular, shape, as depicted exemplarily in Fig. 3B. Optionally, all of the circular, or substantially circular, perforations may exhibit a diameter of 3 mm.

In a yet further specific embodiment, the cross-sectional area (Aₘ) of the main fluid channel (5) ranges from about 530 mm² to about 760 mm²; the cross-sectional area (A_{b}) of the bypass fluid channel (12) ranges from about 1 mm² to about 4 mm²; and
the perforated disk (8) exhibits from about 30 to about 70 perforations (8.1) with a circular, or substantially circular, or regular polygonal shape, and a total combined area of all perforations (8.1) from about 30 % to about 50 % of the cross-sectional area of the main fluid channel (5) at the position of the perforated disk (8).

For instance, in one of the preferred embodiments, the cross-sectional area (Aₘ) of the main fluid channel (5) is in the range of about 550 mm² to about 630 mm², such as about 587 mm²; the cross-sectional area (A_{b}) of the bypass fluid channel (12) may be selected within the range of about 1 mm² to about 4 mm², such as from about 1 mm², or about 1.5 mm², or about 2 mm², or about 2.5 mm², or about 3.2 mm², or about 4 mm², and may optionally exhibit a rectangular, or substantially rectangular, cross-section (e.g. quadratic, or substantially quadratic); and the perforated disk (8) exhibits from about 35 to about 50 circular, or substantially circular, perforations (8.1) and a total combined area of all perforations (8.1) from about 40 % to about 50 % of the cross-sectional area of the main fluid channel (5) at the position of the perforated disk (8).

In one of the further preferred embodiments, the the cross-sectional area (Aₘ) of the main fluid channel (5) is in the range of about 550 mm² to about 630 mm², such as about 587 mm²; the cross-sectional area (A_{b}) of the bypass fluid channel (12) is selected from about 1 mm² or about 4 mm²; and the perforated disk (8) exhibits from about 35 to about 50 perforations (e.g. 37) circular, or substantially circular, perforations (8.1) and a total combined area of all perforations (8.1) of from about 40 % to about 50 % (e.g. about 45 %) of the cross-sectional area of the main fluid channel (5) at the position of the perforated disk (8).

With regard to the `perforated area' (Aₚ) of the perforated disk (8), it should be understood that this area also depends on - and/or is to be adjusted in relation to - the dimensions of the bypass fluid channel (12), such as it cross-sectional area (A_{b}), and more specifically, the cross-sectional area (A_{b}) of the bypass fluid channel (12) with respect to the cross-sectional area (Aₘ) of the main fluid channel (5). If e.g. the cross-sectional area (A_{b}) of the bypass fluid channel (12) is larger, more air may be redirected there; so, the perforated disk (8) should exhibit a larger `perforated area' (Aₚ) as well. In one embodiment, the ratio of the `perforated area' (Aₚ) of the perforated disk (8) to the cross-sectional area (A_{b}) of the bypass fluid channel (12) ranges from about 150 to about 350, such as 250. However, it should be understood, that the exact ratio of the `perforated area' (Aₚ) of the perforated disk (8) to the cross-sectional area (A_{b}) of the bypass fluid channel (12) is of lower relevance as long as the perforated disk (8) causes a fluid flow in the bypass fluid channel (12) which is from about 1: 2.5 to about 1 : 200, or from about 1: 4.5 to about 1: 200, or from about 1: 10 to about 1: 200 of the fluid flow in the main fluid channel (5), and/or ranging from about 0.3 SLM to about 350 SLM, or from about 0.3 SLM to about 200 SLM, or from about 0.3 SLM to about 90 SLM, as mentioned above.

The perforated disks (8) may be prepared by any technique suited to provide perforations of the desired shape and size which is needed for providing a flow resistance, or impedance, in the range from about 0.01 to about 0.15 kPa/(L/s); and/or to cause a fluid flow in the bypass fluid channel (12) which is from about 1: 2.5 to about 1 : 200, or from about 1 : 4.5 to about 1 : 200, or from about 1 : 10 to about 1: 200 of the fluid flow in the main fluid channel (5). This can be achieved for instance by cutting or die-cutting the perforations (8.1) into the disk (8) using e.g. a laser cutter or water jet cutter, a die cutter, a punch, or the like. Alternatively, the disk (8) may be molded or otherwise 'positively' formed, such as by 3D-printing techniques. In other words, the term 'perforation' is used herein synonymously to 'opening', or `hole' or the like, and is not intended to imply a specific preparation method which necessarily involves cutting, punching or stamping or similar techniques which form the perforations by removing material from the blank disk.

In order to advantageously allow molding the perforated disk (8) as one single piece, it was modified in comparison to those used in e.g. industrial gas flow measurement applications. In one embodiment, the perforated disk (8) is provided with a width, or thickness, of about 1 mm to about 4 mm. In a further embodiment, the perforated disk (8) is molded or 3D-printed and exhibits a width, or thickness, of about 1 mm to about 4 mm. In a yet further embodiment, the perforated disk (8) exhibits a width, or thickness, of about 1 to 4 mm, a total surface area in the range of of from about 550 mm² to about 630 mm², such as about 587 mm² and 35 to 50 perforations (8.1), e.g. 37,; and a `perforated surface area' (Ap) of about 40 to 50 % (e.g. 45 %) of the perforated disk's (8) total surface area.

In one embodiment, the distance between the perforated disk (8) and the first lateral opening (6) along the longitudinal axis of the main fluid channel (5) of the spirometer (1) is from about 5 mm to about 15 mm, and preferably from about 8 mm to about 12 mm, e.g. 10.0 mm; and the distance between the perforated disk (8) and the second lateral opening (7) from about 25 mm to about 45 mm, and preferably from about 30 mm to about 40 mm, e.g. 34.2 mm. However, it should be understood, that the exact spacing of the perforated disk (8) between the first and second lateral opening (6 and 7) is of lower relevance as long as the perforated disk (8) causes a fluid flow in the bypass fluid channel (12) which is from about 1 : 2.5 to about 1 : 200, or from about 1 : 4.5 to about 1: 200, or from about 1: 10 to about 1: 200 of the fluid flow in the main fluid channel (5), and/or ranging from about 0.3 SLM to about 350 SLM, or from about 0.3 SLM to about 200 SLM, or from about 0.3 SLM to about 90 SLM, as mentioned above.

According to invention, the spirometer (1) is equipped with a MEMS-based thermal flow sensor (13) for generating a signal in response to the fluid flow in the bypass fluid channel (12). For this purpose, both the MEMS-based thermal flow sensor (13) and the bypass fluid channel (12) are part of, or housed within, the spirometer's (1) main body (9), and the MEMS-based thermal flow sensor (13) is positioned at the bypass fluid channel (12).

In one of the preferred embodiments, the MEMS-based thermal fluid flow sensor (13) is arranged centrally on one of the transverse axis of the bypass fluid channel (12) which is perpendicular to one of the longitudinal axis of the bypass fluid channel (12). For instance, in a specific embodiment, the bypass fluid channel (12) has a quadratic, or substantially quadratic, cross-section of 1 x 1 mm or 2 x 2 mm, and the MEMS-based thermal fluid flow sensor (13) has a side length or - depending on the sensor's shape - a diameter of 0.7 mm, and the MEMS-based thermal fluid flow sensor (13) is positioned centrally in such a way as to have a distance to each of the two neighbouring side walls of the bypass fluid channel (12) of 0.15 mm or 0.65 mm, respectively. Such central positioning is believed to be an important contribution in the task of providing the high precision, reproducibility and sensitivity observed with the spirometer (1) according to the first aspect of the invention when compared to prior art portable spirometers.

Optionally, and in one of the preferred embodiments, the MEMS-based thermal fluid flow sensor (13) may be positioned such as to be flush with the bypass fluid channel (12) wall through which it reaches into the 'lumen' of said channel (12) and comes in fluid connection with said 'lumen' and the fluid flow passing through it. In other words, the bypass fluid channel (12) exhibits an opening in its lateral walls into which the the MEMS-based thermal fluid flow sensor (13, 13.1, 13.2) is placed such as to be flush with the lateral walls of the bypass fluid channel (12), or at least flush with the lateral walls of the bypass fluid channel (12) directly surrounding said opening. Positioning the MEMS-based thermal fluid flow sensor (13) such as to be flush with the lateral walls of the bypass fluid channel (12) is preferred in that it offers the advantage that the sensor interferes the least with the laminar fluid flow throught the bypass fluid channel (12), and thus improves sensitivity and accuracy.

Alternatively, the MEMS-based thermal fluid flow sensor (13) may reach further into the 'lumen' of the bypass fluid channel (12); for instance, such as to be positioned approximately at the central longitudinal axis of the bypass fluid channel (12) and about equidistant to all surrounding bypass fluid channel (12) walls.

Further optionally, the MEMS-based thermal fluid flow sensor (13) may be arranged centrally both on one of the transverse axis of the bypass fluid channel (12) which is perpendicular to one of the longitudinal axis of the bypass fluid channel (12), and centrally on one of the longitudinal axis of the bypass fluid channel (12).

In one embodiment, the MEMS-based thermal fluid flow sensor (13) of the spirometer (1) is a bidirectional flow sensor (13.1), such as to allow e.g. for measurements during both inhalation and exhalation. In this configuration, the MEMS-based thermal fluid flow sensor (13, 13.1) enables the determination of all relevant spirometry parameters: FVC, FEV1, FEV_{1%}, PEF, FEF_{25-75%}, FET, EVOL, ELA, VC, IVC, IC, ERV, FEV₁/FVC_{%}, FEV_{0.5}, FEV_{0.5}/FVC%, FEV_{0.75}, FEV_{0.75}/FVC%, FEV₂, FEV₂/FVC%, FEV₃, FEV₃/FVC_{%}, FEV₆, FEV₁/FEV_{6%}, FEF_{25%}, FEF_{0.50%}, FEF_{0.75%}, FEF₇₅₋₈₅, FIVC, FIV₁, FIV₁/FIVC_{%}, FIF_{0.25%}, FIF_{50%}. The most commonly evaluated parameters are FVC, FEV, FEV₁, PEF.

In a more specific embodiment, the MEMS-based thermal fluid flow sensor (13) is a monolithic CMOS flow sensor (13.2; complementary metal-oxide-semiconductor) comprising a sensor chip, the chip comprising an encapsulated gas bubble, a microheater for heating the gas bubble, a first plurality of thermopiles located on a first side of the gas bubble, and a second plurality of thermopiles located on a second side of the gas bubble which is opposite to the first side (e.g. referred to as the upstream and downstream sides, respectively). The thermopiles may, for instance, be aluminum/polysilicon thermopiles.

This specific type of MEMS-based thermal fluid flow sensor (13), using electric thermopiles, operates via thermoelectric transduction and allows a differential measurement: under zero fluid flow, the temperature is the same at both the upstream thermopiles and the downstream thermopiles, causing both to output the same voltage. Under non-zero fluid flow, the velocity of the fluid flow disturbs the temperature profile across the micro heater, causing a heat asymmetry or temperature gradient, thus causing the voltage output of the the upstream thermopiles and the downstream thermopiles to be different. Using such electric transduction differential measurement and allows to have excellent sensitivity and unbiased output voltage with no offset or drift; this is required for reliable measurements of all above-mentioned relevant spirometry parameters. In contrast, most other 'calorimetric type flow sensors' (e.g. thermoresistive sensors operating on measuring the resistance of the resistor while cooled down by air flow) are unsuitable for use in the spirometer (1) of the invention.

In a further specific embodiment, the MEMS-based thermal fluid flow sensor (13) is a monolithic CMOS flow sensor (13.2; complementary metal-oxide-semiconductor) comprising a sensor chip, the chip comprising an encapsulated gas bubble, a microheater in the form of a heating rod comprising a plurality of dynamically controlled thermal resistors for heating the gas bubble, a first plurality of thermopiles arranged in rows and located on a first side of the gas bubble, and a second plurality of thermopiles arranged in rows and located on a second side of the gas bubble which is opposite to the first side. Again, the thermopiles may, for instance, be aluminum/polysilicon thermopiles.

In a yet further specific embodiment, the MEMS-based thermal fluid flow sensor (13) is a bidirectional monolithic CMOS flow sensor (13.1, 13.2) comprising a sensor chip, the chip comprising an encapsulated gas bubble, a microheater in the form of a heating rod comprising a plurality of dynamically controlled thermal resistors for heating the gas bubble, a first plurality of thermopiles arranged in rows and located on a first side of the gas bubble, and a second plurality of thermopiles arranged in rows and located on a second side of the gas bubble which is opposite to the first side. In a specific embodiment, the heating rod comprises four dynamically controlled thermal resistors for heating the gas bubble. The thermopiles may, for instance, be aluminum/polysilicon thermopiles.

One of the benefits of employing this type of MEMS-based thermal fluid flow sensors (13) with a microheater in the form of a heating rod comprising a plurality of dynamically controlled thermal resistors (e.g. four thermal resistors) for heating the gas bubble, is that when the fluid flow in the bypass fluid channel (12) increases, the heater power will increase accordingly until the heater power is saturated. Due to the plurality of dynamically controlled thermal resistors, the MEMS-based thermal fluid flow sensors (13, 13.1, 13.2) may also be used over a very wide range of environmental conditions, for instance, 0 - 3000 meters above sea level and in temperatures ranging from -5 °C to + 40 °C. In addition, Due to the plurality of dynamically controlled thermal resistors, the MEMS-based thermal fluid flow sensors (13, 13.1, 13.2) may also be used over a wider range of flow rates because this plurality of resistors allows a 4-6-fold increase in the measurement range; for instance, from a MEMS-based thermal fluid flow sensor having just one thermal resistor and a measurement range of about 0-200 SLM to a range of about 0-800 SLM, or even 0-1000 SLM with four thermatl resistors. Furthermore, employing this type of MEMS-based thermal fluid flow sensors (13) with the thermopiles arranged in rows, or series, significantly increases the accuracy as well as measurement range. The thermopiles may, for instance, be aluminum/polysilicon thermopiles.

In a preferred embodiment, the thermopiles (e.g. aluminum/polysilicon thermopiles) are symmetrically positioned upstream and downstream of the micro-heater, preferably in flow-direction (i.e. parallel to the direction of fluid flow), such that in the presence of fluid flow, or gas flow, the thermopiles will show temperature differences from which a) the fluid flow may be calculated, and b) the exhale temperature can be determined; i.e. such a monolithic CMOS flow sensor (13.2) also acts as a breath temperature sensor (26). The sensor chip can be mounted on a printed circuit board along with e.g. the microcontroller (14) as depicted in figure 4.

In one of the further preferred embodiments, the MEMS-based thermal fluid flow sensor (13) is a bidirectional monolithic CMOS flow sensor (13.1, 13.2) comprising a sensor chip with an encapsulated gas bubble as described above; and the bypass fluid channel (12) has a rectangular, or substantially rectangular (e.g. a quadratic, or substantially quadratic) cross section, and is adapted in such a way that the width of the lateral wall of said bypass fluid channel (12) through which the MEMS-based thermal fluid flow sensor (13) gets in fluid connection with the 'lumen' of the bypass fluid channel (12), at the position of the MEMS-based thermal fluid flow sensor (13), is only a few millimetres larger, more specifically only about 0.1 to 6 mm larger, than the MEMS-based thermal fluid flow sensor (13); preferably only about 0.2 to 4 mm larger; more preferably only about 0.3 to 3 mm larger. In an exemplary embodiment, the MEMS-based thermal fluid flow sensor (13) exhibits a width of 0.8 mm; and the lateral wall of the bypass fluid channel (12) through which the MEMS-based thermal fluid flow sensor (13) gets in fluid connection with the 'lumen' of the bypass fluid channel (12), at the position of the MEMS-based thermal fluid flow sensor (13) exhibits a width of 1.0 mm; thus, allowing the gas bubble within the sensor to interact with the fulid flow in an optimized way and thus maximize precision of the measurements.

In a specific embodiment, the communication of the MEMS-based thermal fluid flow sensor (13, 13.1, 13.2) with the microcontroller (14) is achieved via a so-called SPI bus (serial peripheral interface).

The MEMS-based thermal fluid flow sensor (13, 13.1, 13.2) - or hereafter shortly referred to as the flow sensor (13, 13.1, 13.2) - is positioned at the bypass fluid channel (12) for generating a signal in response to the fluid flow in the bypass fluid channel (12). As mentioned, the bypass fluid channel (12) extends from the first to the second fluid opening (10 and 11), and therefore - as long as the tubular mouthpiece (2) and the main body (9) of the spirometer (1) are attached to each other - also from the first to the second lateral opening (6 and 7) of the tubular mouthpiece (2), such that a fluid communication between the main fluid channel (5) and the bypass fluid channel (12) is provided. In one embodiment, the bypass fluid channel (12) has a parallel orientation to and extends over a longitudinal portion of the main fluid channel (5). This may be seen e.g. in figure 2.

In one embodiment, the spirometer (1) further comprises an acceleration sensor (15) which is different from the flow sensor (13, 13.1, 13.2), as shown for instance in figure 4. In other words, the portable, handheld electronic spirometer (1) of this embodiment comprises:
(a) a tubular mouthpiece (2) with a proximal opening (3) for insertion into the mouth of a user, a distal opening (4), a main fluid channel (5) extending between the proximal opening (3) and the distal opening (4) and exhibiting a cross-sectional area (Aₘ) in the range of from about 200 mm² to about 1400 mm², a first lateral opening (6), a second lateral opening (7) positioned at a longitudinal distance to the first lateral opening (6), and a flow restrictor in the form of a perforated disk (8) positioned in a cross-sectional orientation in the main fluid channel (5) between the first and the second lateral opening (6 and 7); and
(b) a main body (9) with a first fluid opening (10) connectible with the first lateral opening (6) of the mouthpiece (2), a second fluid opening (11) connectible with the second lateral opening (7) of the mouthpiece (2), a bypass fluid channel (12) extending between the first and the second fluid opening (10 and 11) and exhibiting a cross sectional area (A_{b}) in the range of from about 1 mm² to about 16 mm², a MEMS-based thermal fluid flow sensor (13, 13.1, 13.2) positioned at the bypass fluid channel (12) for generating a signal in response to the fluid flow in the bypass fluid channel (12), an acceleration sensor (15, 15.1) which is different from the MEMS-based thermal fluid flow sensor (13, 13.1, 13.2), a microcontroller (14) connected with the fluid flow sensor (13, 13.1, 13.2) for calculating the fluid flow from the signal generated by the flow sensor (13, 13.1, 13.2), a communication means for the exchange of data related to the fluid flow generated by the micro-controller (14) of the spirometer (1);
wherein i) the cross-section of the bypass fluid channel (12) with respect to the cross-section of the main fluid channel (5), and ii) the perforated disk (8) are adapted, or configured, in a such way that the flow resistance of the spirometer (1) does not exceed 0.15 kPa/(L/s).

It should be understood, that this acceleration sensor (15) is preferably incorporated within and/or an integral part of the spirometer (1), usually as part of the spirometer's main body (9), e.g. on the printed circuit board; in other words, the acceleration sensor (15) is not provided separate or external from the spirometer (1). This set-up is selected to ensure that, while being different from the flow sensor (13, 13.1, 13.2), the acceleration sensor (15) is still exposed to the same or very similar external influences (such as temperature, movement, vibration, etc.) as the flow sensor (13, 13.1, 13.2); and/or, to ensure that the sensitivity achieved is matching the sensitivity needed for high precision spirometry. Same as the flow sensor (13, 13.1, 13.2), this acceleration sensor (15) is directly or indirectly connected with the microcontroller (14) such that the microcontroller (14) is capable of receiving a signal from the acceleration sensor (15). The acceleration sensor (15) can, for instance, be mounted on a printed circuit board along with e.g. the flow sensor (13, 13.1, 13.2) and the microcontroller (14) as depicted in figure 4. However, unlike the flow sensor (13, 13.1, 13.2), this acceleration sensor (15) is not connected to the main fluid channel (5) or the bypass fluid channel (12), such as to generate signals which are predominantly related to vibrations, or noises, caused by movements, or accelerations, of the spirometer (1).

The flow restricting perforated disk (8) in the above described preferred embodiment may be any one of the perforated disks (8) described earlier, preferably a perforated disk (8) comprising from about 15 to about 100 perforations (8.1), or form about 30 to about 70 perforations (8.1); for instance, a perforated disk (8) with a total surface area in the range of from about 550 mm² to about 630 mm², such as about 587 mm² and
from about 40 to about 70 hexagonal perforations (8.1) (e.g. 55) with a `perforated surface area' (Ap) of from about 160 mm² to about 205 mm² (e.g. about 175 mm²), or from about 28 % to about 35 % (e.g about 30 %) of the disk's total surface area; or
from about 35 to about 50 perforations (e.g. 37) circular, or substantially circular,
perforations (8.1) with a `perforated surface area' (Ap) of about from about 235 mm² to about 285 mm² (e.g. 262 mm²), or from about 40 % to about 50 %(e.g. about 45 %) of the disk's total surface area.

As mentioned, the flow sensor (13, 13.1, 13.2) is rather sensitive to vibrations, or noises, resulting e.g. from a movement or acceleration of the spirometer (1). Therefore, an additional acceleration sensor (15) which is not connected to the main fluid channel (5) or the bypass fluid channel (12), but incorporated within the spirometer (1), in particular within the main body (9) of the spirometer (1), allows for the correction of the calculated fluid flow in that they detect such non-flow-related vibrations, or noises, and enable the subtraction of it from the fluid flow signal generated by the flow sensor (13, 13.1, 13.2), and/or allow for the verification that a measurement of the flow sensor (13, 13.1, 13.2) was performed under suitable conditions (such as without significant noise and/or without linear accelarations as the user may cause by moving during breathing manoeuvers); and, as the case may be, to guide the user of the spirometer (1) to repeat an unsuitable measurement. In other words, the acceleration sensor (15) is used to confirm the quality of the spirometric measurements by either allowing a correction the calculated fluid flow values (e.g. in case of smaller and regular movements as they typically occur when users, or patients, breath through the spirometer), whereas for larger, more forceful movements, the acceleration sensor (15) would help to alert the user, or patient, to repeat the breathing manoeuver with less movement.

In one embodiment, the microcontroller (14) of the spirometer (1) is programmed to calculate a corrected fluid flow from the signal generated by the flow sensor (13, 13.1, 13.2) and from a signal generated by the acceleration sensor (15). In a specific embodiment, the microcontroller (14) is connected both with the fluid flow sensor (13, 13.1, 13.2) and the acceleration sensor (15, 15.1) and is programmed to calculate a corrected fluid flow from the signal generated by the flow sensor (13, 13.1, 13.2) and from a signal generated by the acceleration sensor (15,15.1).

In a more specific embodiment, the acceleration sensor (15) is a 3-axis sensor (15.1) with a sensitivity (So) of at least 973 counts/g ± 5 % for each of the three axes; typically, the sensitivity ranges between 973 and 1075 counts/g; e.g. 1024 counts/g; for instance, a MMA8491QR1 unit as supplied by Freescale Semidconductors. This MMA8491QR1 unit is a low voltage, multifunctional digital 3-axis, 14-bit ± 8 g accelerometer housed in a 3 x 3 mm casing and may communicate with the microcontroller (14) via a common inter-integrated circuit bus (I²C bus), or I²C interface. It covers an acceleration range of ± 8 per axis and data may be read from the sensor with 1 mg/LSB sensitivity.

It was surprisingly found that the use of a MEMS-based thermal fluid flow sensor (13, 13.1, 13.2) in the spirometer (1) of the invention as claimed, together with an incorporated acceleration sensor (15, 15.1) and a perforated disk (8) as a flow restrictor, provides a remarkably high precision to the inventive spirometer (1) In fact, it renders the device sensitive enough to even measure the minute movements of air moved in or out of the trachea by the heart beat, thus enabling not only full spirometry as intended but also new medical uses which were not available before with prior art spirometers; for instance, high precision full spirometry in connection with the possibility to monitor the heart beat frequency of a patient simultaneously. According to the knowledge of the inventors, such high precisions could not be achieved in the past with prior art portable devices which are evaluating the fluid flow by measuring either a pressure difference before and after a flow restrictor with a known resistance (e.g. using a differential pressure sensor), or by the rotations of a turbine.

In addition, the device may be manufactured easily and at low manufacturing costs, allowing to offer a low-priced, lightweight, energy-efficient, yet highly precise portable, handheld electronic spirometer (1), which does not require large and/or heavy energy sources.

In one embodiment, the acceleration sensor (15, 15.1) is further employed for measuring the temperature of the breath; similar to the MEMS-based thermal fluid flow sensor (13, 13.1, 13.2).

In one embodiment, the electronic spirometer (1) further comprises a gyroscope (29), preferably in addition to the acceleration sensor (15, 15.1); or in other words, the electronic spirometer (1) further comprises a gyroscope (29) which is different from the MEMS-based thermal fluid flow sensor (13, 13.1, 13.2) and the acceleration sensor (15, 15.1). The gyroscope (29) detects the horizontal orientation of the spirometer (1) and can be used to detect non-perpendicular orientation of the device during a spiroemtric measurement manoeuvre (e.g. if the user, or patient, would bend the head down to the chest during a breathing manoeuvres). This allows for automatically alerting the user to correct his/her position, and thus for further improved quality of the single manoeuvre as well as the longterm analysis of lungfunction parameters; in particular, of unsupervised and/or laypersons' spirometry manoeuvres. Similar, to the acceleration sensor (15, 15.1), also the gyroscope (29) allows for the verification that a measurement of the flow sensor (13, 13.1, 13.2) was performed under suitable conditions (such as without significant deviations from holding the spirometer (1) approximately horizontal), and as the case may be to guide the user of the spirometer (1) to repeat an unsuitable measurement.

In that regard, both the acceleration sensor (15, 15.1) which is different from the flow sensor (13, 13.1, 13.2) and/or the gyroscope (29) can each be understood as part of an 'in-built quality control system' which facilitates more reliable and accurate spirometric flow measurements and hence makes the spirometer (1) according to the invention particularly suited to laypeople users.

In one embodiment, the spirometer (1) further comprises a heart rate sensor (16), a blood oxygen saturation sensor (17; also called pulse oximetry sensor or SpO2 sensor), a temperature sensor for measuring the temperature of the environment (18), an atmospheric pressure sensor (19), and/or a moisture sensor (20; also called humidity sensor). Each of these one or more sensors (16-20) is directly or indirectly connected with the microcontroller (14) such that the microcontroller (14) is capable of receiving a signal from each of the one or more sensors (16-20).

In one embodiment, the heart rate sensor (16) and the blood oxygen saturation (17) are contained within one and the same sensing means, i.e. a combined sensor as depicted e.g. in figure 2. In a specific embodiment, this combined sensor operates by reflecting light waves of two distinct wavelengths - usually red (about 600-750 nm) and infrared (about 780 nm -1 mm) - from a vascularized tissue and measuring the remitted light (i.e. reflected or scattered) with a recipient photodiode. Typically, these combined sensors allow two operation modes: SpO2 (red and infrared diodes switched on interchangeably) or heart rate only (only infrared diode switched on). In a more specific embodiment, the combined heart rate and blood oxygen saturation (16, 17) is a MAX30100 module as supplied by Maxim Integrated. The system comprises a red diode, an infrared diode and a photodiode, as well as filtering blocks and digital signal processing units including a I²C (TWI) digital interface. Communication with the sensor allows to control the sampling parameters and current of both light diodes, providing the possibility of dynamically correcting the amplitude of the output signal. Sampling frequencies range within50 Hz to 1 kHz, corresponding to illuminating times of the diodes from 200 µs to 1600 µs.

Optionally, the blood oxygen saturation (17) - or the combined heart rate and blood oxygen saturation (16, 17) - is housed in the spirometer's (1) main body (9) in such a way that the user's fingers naturally cover the blood oxygen saturation (17) while holding the spirometer (1) in hand during the inhalation and/or exhalation manoeuvres, as is depicted in figure 2.

In one embodiment, the spirometer (1) comprises all three environmental sensors, namely the temperature sensor, the atmospheric pressure sensor and the moisture sensor (18-20). In a more specific embodiment, one or all of these environmental sensors (18-20) are supplied with 3.3 V and communicate with the microcontroller (14) via a common I²C bus.

In one embodiment, the temperature sensor (18) and the humidity sensor (20) are contained within one and the same sensing means; i.e. a combined sensor as depicted in figure 4. In a specific embodiment, the combined sensor is a digital sensor SHT21D (version 3) as supplied by Sensirion which allows sampling frequencies up to 2 Hz with 12-bit measurement resolution.

In one embodiment, the atmospheric pressure sensor (19) is selected from any sensor capable of measuring pressures in at least the range of about 800 hPa to about 1100 hPa, or about 0.8 bar to about 1.1 bar; preferably sensors which are especially designed for mobile applications, such as piezo-resistive pressure sensors. In a specific embodiment, the atmospheric pressure sensor (19) is a digital BMP280 sensor as supplied by Bosch.

The positioning of the three environmental sensors (18, 19, 20) on the main board (27) is depicted in figure 4. As mentioned earlier, these environmental sensors (18, 19, 20) may be used e.g. for the BTPS conversion of FVC measurements (BTPS: body temperature pressure saturated); i.e. the vital capacity at maximally forced expiratory effort, expressed in litres at body temperature and ambient pressure saturated with water vapour, as required by the ATS standards of spirometry in order to allow for comparability across different temperatures, pressures and humidity conditions; i.e. a standardisation of environmental conditions (see e.g. "Standardisation of spirometry"; Eur Respir J 2005; 26: 319-338). The portable spirometers of the prior art do not have these sensors build in; hence, the user is required to enter these data manually, which makes the device less versatile and increases the risk of erroneous measurements, or data interpretation, in particular with lay-people.

In one embodiment, the microcontroller (14) is provided in the form of a so-called System-on-Chip (SoC) unit on a printed circuit board (PCB) as depicted in figure 4, also referred to as the main board (27). In a specific embodiment, the microcontroller (14) is a nRF51822-QFAC (rev. 3) SoC-unit as obtainable from Nordic Semiconductor and supplied with an ARM Cortex-M0 core which comprising a BLE radio module, a built-in 256 kB flash memory and 32 kB RAM.

According to the invention, the spirometer (1) comprises a communication means, preferably a wireless communication means, and more preferably a radio communication means (21) in order to connect the spirometer (1) to a user's personal computer and/or smartphone or any other computing unit which is adapted to collect, store, analyse, exchange and/or display data. The communication means is employed for the exchange of data related to the fluid flow generated by the spirometer (1), preferably by the microcontroller (14) of the spirometer (1).

The wireless or in particular radio connection can be operative during the measurements, thereby allowing real time display of the measured date. Alternatively, the spirometer (1) may be connected to the user's personal computer and/or his smartphone at a later time point to transfer, or copy, any measured and stored data from the spirometer (1) to the computer and/or smartphone. In a specific embodiment, the radio communication means (21) is a Bluetooth connectivity (21.1), e.g. a Bluetooth 4.0 connectivity. In a further specific embodiment, the radio communication means (21) is a so-called Near Field Communication (NFC) means (21.2) or a Wireless Local Area Network (WLAN) means (21.3). Optionally, different types of radio communication means (21) may be combined in a device, e.g. a Bluetooth connectivity (21.1) together with an NFC means (21.2), as depicted on the main board (27) of figure 4.

Measured parameters are digitalized and then wirelessly transmitted to the user's personal computer and/or smartphone or any other computing unit which is adapted to collect, store, analyse, exchange and/or display data, optionally via one or more remote data servers, also called 'cloud'. With regard to the cloud it should be understood, that unlike other prior art devices, the spirometer (1) of the invention may also use a cloud, but does not require it for the device to be operable, to perform the measurement(s) and/or to obtain the result; all computing is done locally on the smartphone.

Further alternatively, or in addition to the radio communication means (21, 21.1, 21.2, 21.3), the spirometer (1) may further comprise a cable communication means (22) via a serial bus, such as a USB connection (22.1).

Both these communication means (wireless or using a cable connection) may further be employed for firmware updates.

In one embodiment, the spirometer (1) further comprises a RAM (random-access memory) and a flash memory in order to store measured data.

As mentioned, the spirometer (1) may be connected to a user's personal computer and/or his smartphone, for analysis, visualisation and also storage of the measured spirometry data; preferably via a dedicated spirometer application ('app') using a proprietary and predictive medical algorithm; or as an 'add-on' integrated in other existing healthcare apps available for iOS or android phones, such as GoogleFit, HealthKit, CareKit or the like (i.e. apps intended as personal and central data collection points for connected third-party electronic accessories for medical and general fitness purposes, where users can e.g. create a medical ID with important medical details).

The dedicated proprietary app is employed to receive signals from the spirometer (1), measure and analyse results in real-time, display the appropriate parameters, store past results, provide diagnostic support, generate printabe files (e.g. PDF) for keeping a paper/computer format log, and optionally to send results to physicians. With the help of the portable, handheld electronic spirometer (1) and the related app, users can thus track their personal respiratory parameters, as well as the responsiveness to and adequacy of medication, in a far more close-meshed manner than achievable in e.g. hospital settings.

In one embodiment, the collected data (up to 1,000,000 results) from the spirometer (1) are stored in the form of a logged history in the local, internal database of the app such that the data are readily available for the user even when he/she is offline. In case a user uninstalls the application, the database is also removed; however, Android's backup and iOS's CloudKit services allows users to copy persistent application data to remote cloud storage in order to provide a restore point for the application data and settings. When performing a factory reset or converting to a new device, the system automatically restores backup data when the app is re-installed, such that users don't need to reproduce their previous data or settings. Alternatively, or in addition to the local storage, cloud storage may be provided as an 'opt-in' option for the user.

Optionally, the data measured and collected with the spirometer (1) may further be combined with geographical data and data of multiple users then analysed collectively on a remote server in order to create maps of specific changes in conditions in a given area and time for e.g. asthmatic users or allergy sufferers. The data collected via such geolocation provides a framework for building analytical knowledge, correlating data to a certain area, and - where considered expedient - for providing this knowledge to users and/or doctors in a given area, e.g. in the form of alerts on upcoming acute exacerbations and/or increased allergy risks sent to their personal computers and/or smartphones. This optional functionality will be provided for and to users in an anonymised fashion.

The spirometer (1) may further provide motivational messages to users in order to coach them for self-management. It can also provide instant feedback to the user while performing the spirometry measurement (e.g. audible or visual) which allows instructing and/or promting a user to conduct a desired spirometric breath manoeuvre, such as rapidly exhaling at the right moment. This is believed to be unique for spirometers as other marketed spirometers do not do coach users on how to correctly perform spirometry during the actual measurement, or breath manoeuvre, and/or what to improve in the next manoeuvre. This feedback and/or motivational means facilitates in particular unsupervised use.

In addition, based on data mining and machine learning algorithms comprised in the dedicated app, the spirometer (1) can identify clinical and also environmental patterns (such as temperature, pressure and ambient air humidity) that may be associated with e.g. an upcoming asthma attack and/or a disease progression in a predictive manner. Ultimately, users are thus enabled to eliminate or at least reduce severe hospitalizations due to acute and chronic exacerbations. However, as mentioned, the examination of respiratory parameters may also be helpful for athletes monitoring their training progress or for smokers monitoring the benefits of smoking cessation.

In one embodiment, the spirometer (1) is operated with a long-life battery, such as a lithium-ion poymer (LiPo) battery or a lithium-ion (LiOn) battery. LiPo batteries offer high capacity compared to their small size, and high-speed charging. In a specific embodiment, the battery is a (re)chargeable 3.7V / 300 mAh LiPo battery; for instance, an LP-402933-IS-3 battery featuring a built-in NTC 10 kohm thermistor and a transistor protection against overloading. A low-dropout (LDO) type voltage stabiliser then supplies continuous current of 150 mA and a DC output voltage of 3.3 V when the spirometer (1) is switched on, for instance to the microcontroller (14) and all sensors (13, 15-20). In a specific embodiment, the voltage stabiliser is a TPS706 unit as supplied by Texas Instruments. In addition, a voltage divider may be employed if the voltage directed to certain components of the spirometer (1) cannot exceed specific values; e.g. the voltage sampled by the microcontroller (14) not exceeding 1.2 V.

In one embodiment, the battery is charged via an inductive NFC charging system and/or via a USB or mini-USB connector (22.1). In a specific embodiment, the basic component of the wireless charging module is a 5 W unit (BQ51050B) as supplied by Texas Instruments, charging to the maximum voltage of 4.2 V. A reception coil (Wurth Elektronik coil 760308103205) is connected to the unit with inductiveness of 11 µH. The unit comprises a LiPo and LiOn battery charger with the function of monitoring temperature using an NTC thermistor (10 kohm). It also provides the possibility of selecting a priority for the source of charging; for example, if USB charging is available via a connected mini-USB port, the charging unit will stop wireless charging and switch to USB-charging. In a further specific embodiment, the basic component for the charging module is a BQ24040- unit as supplied by Texas Instruments, a LiPo and LiOn battery charger charging to the voltage of 4.2 V. Maximum charging current is 800 mA, and maximum initial charging by default amounts to 300 mA for devices with a 300mAh battery, such as the battery used in one embodiment of the spirometer (1).

The module which is responsible for detecting the charging source (e.g. wireless vs. USB) also performs the task of automatically starting the spirometer (1) during charging, as is necessary in order to inform the user about the charging status by means of the LEDs (23.1); i.e. the user does not have to start the spirometer (1) manually via ON/OFF button (25) in order to see the charging status. The microcontroller (14) uses the module to check the charging source and status, and this information may also be provided to the user via the app. After charging is completed, the device will be switched off automatically.

In one embodiment, the spirometer's (1) main body (9) is further equipped with optical (23) and/or acoustical (24) signalling means providing use-related information such as ON/OFF-status, battery status and the like to the user. In a specific embodiment, the spirometer's (1) main body (9) is fitted with light emitting diodes (LEDs), for instance a set of blue LEDs (23.1) arranged at the top of the main body (9) as depicted in figure 2. The LEDs display specific status information, such as device start-up, data transfer, low battery (e.g. all diodes flashing) or battery charging status (e.g. diodes illuminating subsequently).

In a more specific embodiment, the direct control of these LEDs is provided by a TLC59108 unit. Each diode consumes only about 5 mA of current (depending on the light intensity) while the microcontroller (14) is able to supply a maximum of about 120 mA. The microcontroller (14) also enables to set the brightness of illumination using a built-in PWM module (pulse-width modulation), as well as to set the mode of diode flashing with particular frequency and duration of illumination on/off-time.

In one embodiment, the spirometer (1) exhibits a mean energy consumption, or current consumption, during its operation that is not higher than about 90 mA in total. Preferably, the mean energy consumption does not exceed about 50 mA, even with all light emitting diodes (LEDs) being illuminated. On average, the spirometer (1) equipped with a freshly charged 300mAh battery is operable for about 120 days in stand-by mode, for about 56 days for single users and for about 5.6 days when used for multiple patients in e.g. a doctor's office. The time estimated for continuous, uninterrupted operation on one battery charge is about 6 h. In other words, the inventive spirometer (1) is not only allowing for spirometric measurements at a remarkably high precision but is highly energy-efficient at the same time, thereby reducing the need for expensive and heavy energy sources.

The spirometer's (1) main components which get in contact with the user's skin, namely the tubular mouthpiece (2) and the main body (9), may be prepared from any bio-compatible material, including biocompatible polymers. In one embodiment, bio-compatible PolyJet photopolymer (MED610) is employed, a rigid medical material suited for prolonged skin contact of more than 30 days and short-term mucosal membrane contact of up to 24 hours, but also suited for rapid prototyping. MED610 features high dimensional stability and colorless transparency. Also polycarbonate-ISO (PC-ISO) may be employed; a high strength thermoplastic material which in its pure form is biocompatible and sterilizable by gamma irradiation or ethylene oxide (sterilization method ET0). PC-ISO is commonly used for packaging medicines and in the manufacture of medical devices.

As mentioned earlier, the front end of the tubular mouthpiece, i.e. the end comprising the proximal opening may optionally be configured as a detachable part of the tubular mouthpiece, thereby allowing to remove this front end portion of the mouthpiece; for instance, to clean it, or to discard and replace it, after contact with a user's lips and/or tongue. In case of such disposable front end portions (or other disposable parts as needed in multi-patient settings), the materials may also include more simple biocompatible materials such as cardboard. Alternatively, or in addition, the detachable front end portion of the mouthpiece may be equipped with one or more filters to remove airborne particles, saliva droplets and/or bacteria; thereby further reducing the risk of contaminating the sensitive MEMS-based thermal fluid flow sensor (13, 13.1, 13.2). Such filter-mouthpieces are available at low cost and thus may be replaced for each patient in multi-patient settings.

Further optionally the spirometer may be provided to the user together with a nose-clip, such as to allow the user to block the nose while performing spirometric measuements. In one embodiment, the nose-clip and the spirometer are provided as a kit, optionally further comprising readable instructions on the correct use of the spirometer and/or the nose-clip.

In a second aspect, the invention provides a method for measuring a lung function associated health parameter of a human subject, the method comprising a step of the human subject performing a breathing manoeuvre through the portable, handheld electronic spirometer (1) as described above. In one embodiment of this method, said health parameter is selected from
a) a forced vital capacity (FVC), b) a forced expiratory volume (FEV) such as the forced expiratory volume in 1 second (FEV1),
c) a peak expiratory flow (PEF), d) a forced expiratory flow (FEF) such as the forced expiratory flow at 25 %-75 % of FVC (FEF25-75), e) a maximum voluntary ventilation (MVV), f) a mean expiratory flow (MEF), g) a slow vital capacity (SVC), h) a maximum speed of expiration, i) a forced inspiratory volume (FIV) such as the forced inspiratory volume in 1 second (FIV1),
i) a forced inspiratory vital capacity (FIVC), k) a peak inspiratory flow (PIF), or any combination of these (e.g. an inspiratory Tiffeneau value: FIV1/FIVC). The actual breathing manoeuvres are the same as performed with prior art spirometers; the specifics will depend on the actual lung function parameter to be determined. Examples may be found in the "Standardisation of spirometry" e.g. as pulished e.g. by the American Thoracic Society (ATS) or the European Respiratory Sociuety (ERS) (see Eur Respir J 2005; 26: 319-338) or the ISO 26782:2009 (specifying requirements for spirometers intended for the assessment of pulmonary function in humans weighing more than 10 kg).

Beyond full spirometry, the spirometer (1) offers further potential applications, or uses, in various clinical scenarios. For instance, the spirometer (1) may be used for the differential diagnosis of dyspnoea; i.e. the device allows differentiating between cardiac vs. respiratory dyspnoea. When patients are admitted to emergency departments due to chest pain and dyspnoea, this is commonly caused by either coronary insufficiency (ischemia), heart failure (lung congestion) or bronchial obstruction (COPD). Commonly, a differential diagnosis is hindered by the significant overlap of about 30 % of ischemic heart disease (coronary artery disease) patients and COPD patients. The spirometer (1) allows to understand if there is a significant obstruction, in which case the spirometric parameters would not be ok. Hence, if the spirometric parameters are ok, while the cardiac parameters are not, the chest pain and further symptoms are most likely of a cardiac origin, while in the vice versa case, the symptoms are most likely caused bronchially. If both, the respiratory and the cardiac parameters are not ok, the chest pain and dyspnea are caused by a combination of either coronary insufficiency (ischemia), heart failure (lung congestion) or bronchial obstruction (COPD).

In that aspect, it should be understood that this type of differential diagnosis would be possible with prior art devices, too; however, the desktop spirometers as commonly found in hospitals are usually rather large and require longer set-up times. The small hand-held spirometer (1) in contrast is far more practical and requires shorter set-up times, rendering it more suitable for use in emergency and/or intensive care units.

Furthermore, the spirometer (1) may be used in hospitals during pre-extubation assessment of respiratory patients which is one crucial element to prevent failed extubations. The spirometer (1) may be used to determine the efficacy of spontaneous breathing of an intubated patient by either applying the flow sensor (13, 13.1, 13.2) directly on the intubation tube and/or by coupling the spirometer (1) with the intubation tube, such as to measure the fluid flow caused by spontaneous breathing while the ventilator is switched off.

Also, evaluation of cardiac arrest patient requires assessment of the electrical activity as well as the haemodynamic function of the heart, the latter usually being evaluated using pulse. However, in patients with peripheral artery disease and/or those with severe peripheral oedema it may be difficult to feel the pulse despite good haemodynamic function of the heart. The spirometer (1) allows to indirectly evaluate the contractions of the heart by sensing the very discreet movements of air in the lungs and trachea which is caused by the heart beats.

In a third aspect, the invention provides a system comprising:
- the portable, handheld electronic spirometer (1) according to the first aspect of the invention, and
- a first air quality measurement device comprising communication means adapted for data exchange with the portable, handheld electronic spirometer (1) and/or with a separate computing unit, and equipped with one or more air quality sensors, prefereably air quality sensors selected from the group consisting of humidity sensors, temperature sensors, atmospheric pressure sensors, MOS-type gas sensors (metal-oxide-semiconductor), airborne-particles sensors, pollen sensors, ozone (O₃) sensors, nitrogen dioxide (NO₂) sensors, sulfur dioxide (SO₂) sensors and carbon monoxide (CO) sensors, or other type of gas sensors, for determining determine the air quality at the location of the first air quality measurement device, and optionally
- a separate computing unit adapted to collect and analyse at least the data obtained from the spirometer (1) according to the first aspect of the invention and from the first air quality measurement device.

Air pollution is known to be linked to a decrease in lung function in healthy adults and children, and to adversely impact different acute and chronic pulmonary diseases, such as asthma, chronic obstructive pulmonary disease (COPD), bronchitis and cystic fibrosis (CF). Air pollution can trigger cellular responses in the lung, resulting in cytotoxicity, inflammation, and mutagenesis. Bronchial epithelial cells from patients suffering from pulmonary diseases are highly sensitive to airborne particulate matter-induced oxidative stress and apoptosis at a much lower dose than healthy bronchial cells. Hence, an intense response to the oxidative stress induced by air pollution remains the base for disease progression and exacerbations. This pathomechanism was confirmed in observational studies which showed that the annual average levels of air pollution exposure were associated with lung function decrease and an increased likelihood of exacerbation. Pulmonary exacerbations contribute significantly to the burden of disease, with a negative impact on lung function, quality of life, health system costs.

Especially, particulate matter (PM), pollen, ozone (O₃), nitrogen dioxide (NO₂), sulfur dioxide (SO₂) and carbon monoxide (CO) have been identified as key pollutants impairing health. For instance, there is a close quantitative relationship between increased mortality or morbidity (both daily and over time) and the exposure to high concentrations of inhalable coarse particles (2.5-10 µm; PM10), and inhalable fine particles (≤ 2.5 µm; PM2.5). In fact, PM10 and PM2.5 pollution have health impacts even at very low concentrations; in fact, no threshold has been identified below which no health damage is observed. Therefore, guidelines such as by the World Health Organization (WHO) aim to achieve the lowest possible PM-concentration and advise annual means of max. 10 µg/m³ (PM2.5) or 20 µg/m³ (PM10), and 24-hour means of max. 25 µg/m³ (PM2.5) or 50 µg/m³ (PM10).

Also, excessive ozone (O₃) in the air can have a marked effect on human health. It can cause breathing problems, trigger asthma, reduce lung function and cause lung diseases. In Europe, it is currently one of the air pollutants of most concern. Several European studies have reported that the daily mortality rises by 0.3 % and that for heart diseases by 0.4 %, per 10 µg/m³ increase in ozone exposure. An 8-hour mean of max. 100 µg/m³ is advised by the guidelines.

Epidemiological studies have shown that symptoms of bronchitis in asthmatic children increased in association with long-term exposure to nitrogen dioxide (NOz). At short-term concentrations exceeding 200 µg/m³, it is even toxic, causing significant inflammation of the airways. A 1-hour mean of max. 200 µg/m³ is advised by the guidelines.

Sulfur dioxide (SO₂) can affect the respiratory system and lung function, causing inflammation of the respiratory tract and resulting in coughing, increased mucus secretion, aggravation of asthma and chronic bronchitis and an increased risk of respiratory tract infections. Studies indicate that exposure periods as short as 10 minutes already increase the proportion of asthma patients experiencing changes in pulmonary function and respiratory symptoms. Asthmatic subjects exercising in SO₂-polluted air develop bronchoconstriction within minutes, even at levels as low as 0.25 ppm. Lung function parameters such as FEV1 were decreased in response to exposure to only 0.4 to 1.0 ppm SO₂. Furthermore, hospital admissions due to cardiac disease and mortality are increased on days with SO₂ levels above the recommended 24-hour mean of max. 20 µg/m3 or the recommended 10-minute mean of max. 500 µg/m³.

Carbon monoxide (CO) remains the second most strongly correlated air pollutant causing asthma hospital admissions.

The first air quality measurement device is used to generate data related to the air quality (or lack thereof), for instance, the nature and/or the extent of air pollutants (ozone, pollen, particulate matter, etc.) present at any given time at the location of the first air quality measurement device, such as inside the home of the subject using the spirometer (1).

For this purpose, the first air quality measurement device comprises one or more sensors selected from the group consisting of humidity sensors, temperature sensors, atmospheric pressure sensors, MOS-type gas sensors (metal-oxide-semiconductor), airborne-particles sensors, pollen sensors, ozone (O₃) sensors, nitrogen dioxide (NO₂) sensors, sulfur dioxide (SOz) sensors, carbon monoxide (CO) sensors and other type of gas sensors. These sensors may be provided separately (in other words, one sensor for each measurand). Alternatively, the sensors may be combined such as to use one sensor for a plurality of measurands. Examplary and non-limiting embodiments of these sensors will be described below.

In one embodiment, the humidity sensor, the temperature sensor and the pressure sensor may be provided in combined form. In a specific embodiment, the sensor is a Bosch^{®} BME280 sensor, a small (2.5 x 2.5 x 0.93 mm), high performance combined, digital humidity-, pressure, -and temperature sensor with a low power consumption. The humidity sensor provides an extremely fast response time and high overall accuracy over a wide temperature range. The pressure sensor is an absolute barometric pressure sensor with extremely high accuracy and resolution and drastically lower noise. The integrated temperature sensor has been optimized for lowest noise and highest resolution. Its output is used for temperature compensation of the pressure and humidity sensors and can also be used for estimation of the ambient temperature.

In one embodiment, the MOS-type gas sensor is a FIGARO^{®} TGS8100 air quality sensor comprising a sensing chip with a metal-oxide semiconductor (MOS) layer and an integrated heater on a silicon substrate. The sensor is housed in a standard surface-mount ceramic package and requires a heater power consumption of only 15 mW. In the presence of detectable gases (such as hydrogen, ethanol, carbon monoxide (CO), isobutane, methane, cigarette smoke, kitchen odors, or the like), the sensor conductivity increases depending on gas concentration in the air. A simple electrical circuit can convert the change in conductivity to an output signal which corresponds to the gas concentration.

In one embodiment, the airborne-particles sensor is a Sharp^{®} GP2Y1030AU0F, a high sensitivity airborne-particles sensor (also called dust sensor) operating with a built-in microcomputer and an optical sensing system that can detect e.g. particulate matter like PM2.5 and PM10. An infrared emitting diode (IRED) and a phototransistor are diagonally arranged in the sensor to detect the light reflected by airborne particles such as dust and/or cigarette smoke with the sensor being able to distinguish these two by a pulsed pattern of output voltage.

In one embodiment, the ozone (O₃) sensor is a small-sized (15 x 15 x 3 mm), printed ozone sensor, such as the 3SP-O3-20 sensor by SPEC sensors.

In one embodiment, the nitrogen dioxide (NO₂) sensor is an electrochemical sensor, such as the Figfaro FECS42-20 sensor.

In one embodiment, the sulfur dioxide (SO₂) sensor is an amperometric gas sensor, also provided by SPEC sensors; i.e. an electrochemical sensor which generates a current at a working (or sensing) electrode that is proportional to the volumetric fraction of the SO₂ gas. Besides the working (or sensing) electrode and its counter-electrode, the sensor comprises a reference electrode to improve stability, signal-to-noise ratio, and response time.

In one of the preferred embodiments, the first air quality measurement device is not only responsible for generating data related to the air quality via its inbuilt sensors but further serves as a charging dock, or docking station, for at least the portable, handheld electronic spirometer (1), preferably a Near Field Communication (NFC) charging dock. Like this, the spirometer (1) only needs to be placed on top of the first air quality measurement device to recharge; for instance, over night.

Besides the sensors for determining air quality, the first air quality measurement device further comprises a microcontroller and a communication means, preferably a wireless communication means, and further preferably a Bluetooth connectivity, such as Bluetooth 4.0. In a specific embodiment, the microcontroller is a nRF51422-CEAA as produced by Nordic Semiconductor, comprising a 32-bit ARM^{®} Cortex^{™} M0 central processing unit (CPU) with 256kB flash and 32kB RAM as well as an embedded 2.4 GHz transceiver. The microcontroller allows for both Bluetooth^{®} low energy (BLE; previously called Bluetooth Smart) and ANT^{™} wireless connectivities. A ceramic antenna for Bluetooth 2.4 GHz is used for improved reception and more stable connection.

As mentioned, the separate computing unit is adapted to collect and analyse at least the data obtained from the spirometer (1) and from the first air quality measurement device. The purpose of the separate computing unit is to allow for comparison and/or correlation of the data obtained from the spirometer (1) with the data obtained from the first air quality measurement device (and optionally further data), in order to obtain a deeper insight into e.g. the pathogenesis of respiratory diseases; for instance, to correlate days of poorer results in spirometric lung performance tests run by the spirometer (1) with the air quality data measured by the first air quality measurement device on such days.

For this purpose, the separate computing unit in one embodiment comprises a communication means coupled with a microcontroller for performing data collection and data analysis (e.g. a microcontroller in the form of a so-called System-on-Chip (SoC) unit on a printed circuit board (PCB)); and data storage means (e.g. a random-access memory (RAM) and/or a flash memory) in order to store collected and/or analysed data obtained from at least the spirometer (1) and the first air quality measurement device (hereafter shortly referred to as `spirometer data' and `first air quality data', respectively), and optionally further data.

Furthermore, the separate computing unit typically comprises an interface that is adapted to communicate with a user of the inventive system (e.g. the user of the spirometer (1), his/her physician or caretakers), and to provide information to the user on any of the `spirometer data' and `first air quality data' as well as information obtained from comparing and/or correlating the 'spirometer data' and 'first air quality data'. In one embodiment, this interface is a visual display.

In one embodiment, the separate computing unit comprises a wireless communication means, preferably a radio communication means; e.g. a Bluetooth connectivity or a Near Field Communication (NFC) means.

In one embodiment, the separate computing unit is a personal computer (including laptops and handheld PCs) and/or smartphone.

In a further embodiment, the system may comprise two or mor separate computing units, optionally in the form of personal computers (including laptops and handheld PCs) and/or smartphones.

In one embodiment, the separate computing unit is further communicatively coupled to one or more remote data servers. Said remote servers may be employed to store and analyse the `spirometer data' and 'first air quality data', information obtained from comparing and/or correlating the `spirometer data' and 'first air quality data', and optionally further data.

In one of the preferred embodiments, a proprietary software application (`app') is provided on the separate computing unit and/or the remote date servers for performing the comparison and/or correlation of at least the `spirometer data' and 'first air quality data'. In a specific embodiment, the app may also be programmed to perform further tasks such as displaying the `spirometer data', the 'first air quality data' and/or information obtained from their comparison and/or correlation to a user of the inventive system (e.g. as graphical interpretations of the data via interface(s) of the one or more computing units); monitoring said data and information as well as a user's medication over the course of time; creating printable file formats of any data analysis results; send reminders or warning notice to the user (e.g. related to medication time points, smog-warnings, etc.); and/or sharing information with health care providers such as physicians, care givers, health care oranisations and/or other users of the 'app' (optionally in anonymised form).

Optionally, the system as described above further comprises a nose-clip, such as to allow the user to block the nose while performing spirometric measuements. Further optionally, the system further comprises readable instructions on the correct use of the spirometer and/or the nose-clip.

In one embodiment, the system as described above further comprises a second air quality measurement device adapted for data exchange with the portable, handheld electronic spirometer (1) and/or with a separate computing unit, and equipped with one or more air quality sensors, preferably air quality sensors selected from the group consisting of humidity sensors, temperature sensors, atmospheric pressure sensors, MOS-type gas sensors (metal-oxide-semiconductor), airborne-particles sensors, pollen sensors, ozone (O₃) sensors, nitrogen dioxide (NO₂) sensors, sulfur dioxide (SO₂) sensors carbon monoxide (CO) sensors and other type of gas sensors, in order to determine the air quality at the location of the second air quality measurement device. This second air quality measurement device may be used in addition to the first air quality measurement device, or optionally instead of the first (e.g. when travelling). With respect to the selected sensors, the same provisions may apply as described for the first air quality measurement device described above.

Unlike the first air quality measurement device which is typically more stationary (for instance, set up in the home of the user), the second air quality measurement device may be more easily portable in that it is even smaller and more compact than the first device. For instance, the second air quality measurement device may have a size which allows e.g. attachment to a keychain whereas the first air quality measurement device may have a size and shape resembling an external hard drive (e.g. about 7-17 cm long and about 4-8 cm wide). Like this, the second air quality measurement device may e.g. be used when travelling, or the device may be used at work, in the car or any other place of interest, where the subject using the spirometer (1) wants to determine the air quality. It is also possible to place the second air quality measurement device outside.

In one embodiment, the separate computing unit of the system as described above also collects and analyses the data obtained from the second air quality measurement device. In this case, the data obtained from the second air quality measurement device (shortly, the 'second air quality data') may be treated in the same way as the data obtained from the first device; e.g. compared and/or correlated with the `spirometer data'.

In one embodiment, the separate computing unit further allows for the geolocalisation of at least the air quality data obtained from the first air quality measurement device, and optionally of the air quality data obtained from the second air quality measurement device. The geolocalisation functionality may be provided for all users, preferably in anonymous form such as to retain the privacy of each user. Based on this functionality, the inventive system may be able to, for instance, provide warnings to a user (e.g. on smog, pollen and/or other allergens which may impact their lung functions and/or respiratory health), and/or to create geopgraphic maps of all users along with the changes in their respective lung function and/or respiratory health condition at any given time. The data collected through geolocalisation may thus provide a framework for building an even further analytical knowledge of the data provided, e.g. the correlation to a certain area, to specific wheather phenomena, etc.

This means, by using the system according to the third aspect of the invention, the method according to the second aspect of the invention may be complemented with additional data such as data related to the air quality (pollutants, ozone, etc.) and/or geolocation data, thereby allowing to correlate the health parameter of the human subject (such as FVC, FEV or PEF) with these additional data.

In other words, in a fourth aspect the invention provides a method for measuring one or more lung function associated health parameters of a human subject, the method comprising a step of the human subject performing a breathing manoeuvre through the spirometer (1) as described above as the first aspect of the invention.

In one embodiment, a method for measuring one or more lung function associated health parameters of a human subject is provided, wherein the health parameter is selected from (a) a forced vital capacity (FVC), (b) a forced expiratory volume (FEV) such as the forced expiratory volume in 1 second (FEV1), (c) a peak expiratory flow (PEF), (d) a forced expiratory flow (FEF), such as the forced expiratory flow at 25 %-75 % of the FVC (FEF25-75), (e) a maximum voluntary ventilation (MVV), (f) a mean expiratory flow, (g) a slow vital capacity (SVC), (h) a maximum speed of expiration, (i) a forced inspiratory volume (FIV) such as the forced inspiratory volume in 1 second (FIV1), (j) a forced inspiratory vital capacity (FIVC), (k) a peak inspiratory flow (PIF), or any combination of these (e.g. an inspiratory Tiffeneau value: FIV1/FIVC), and
the method comprising a step of the human subject performing a breathing manoeuvre through the spirometer (1) as described above as the first aspect of the invention;
wherein the one or more health parameters are correlated with air quality data, and optionally geolocalisation data, derived from the system as described above as the third aspect of the invention.

### EXAMPLES

### Example 1 - Performance of an exemplary spirometer (1) according to the present invention, equipped with a perforated disk (8.1) for flow restriction

In order to examine the performance of an exemplary spirometer (1) according to the present invention, the device was tested with a flow/volume simulator (here a Series 1120 by Hans-Rudolph; S/N: 122-079) according to the spirometry standards defined by the American Thoracic Society (ATS) and the European Respiratory Society (ERS) as well as by ISO 26782:2009.

A 3D-printed, detachable tubular mouthpiece (2) was provided, comprising a flow-restrictor in the form of a perforated disk (8) with 37 circular, or substantially circular, perforations with 3 mm diameter each, a total surface area of about 587 mm², and a perforated area (Aₚ) of from about 40 % to about 50 % (e.g. about 45 %) of the perforated disk's (8) total surface area. A similar perforated disk (8) is depicted in Fig. 3B. The perforated disk (8) was positioned immovably and in a cross-sectional orientation in the main fluid channel (5) between the first and the second lateral openings (6 and 7) and about perpendicular to the main fluid channel's longitudinal axis.

The bypass fluid channel (12) housed in the spirometer's main body (9) had a quadratic, or substantially quadratic, cross-section of 1 x 1 mm (i.e. cross-sectional area (A_{b}) 1 mm²).

Prior to the actual spirometric measurements, the spirometer (1) was assembled by attaching the tubular mouthpiece (2) to the main body (9). The assembled spirometer (1) was allowed to acclimatise at ambient temperature, pressure and relative humidity in the same room with the flow/volume simulator, and then connected to said simulator for a set of standard calibration tests which involved 64 steady flow waveforms ranging from 0.15 L/s to 18 L/s being discharged from the simulator and through the spirometer (1) and the flow-related signals measured.

Subsequent to calibration, at least the 11 ambient air waveforms, or testprofiles, C1-C11 as defined by the ISO-standard were discharged from the simulator and through the spirometer and FEV₁, FEV₆, PEF and FVC measured three times for each waveform/testprofile. Similarly, the 24 waveforms as defined by ATS-standardization (see e.g. ATS "Standardization of spirometry", Am. J. Respir. Crit. Care Med. 1995; 152: 1107-1136) were discharged from the simulator and through the spirometer and FEV₁ and FVC measured five times for each waveform/testprofile. The results of Example 1 (Ex. 1) will be provided further below.

Comparison Examples 1 to 3 - Performance of the spirometer (1) according to the present invention equipped with other types of flow restrictors.

The same calibrations and measurements as described in Example 1 above were repeated with three almost identical spirometers (1) which differed from the one of Example 1 only in that they comprised different flow restrictors fitted within the 3D-printed, detachable tubular mouthpiece (2) (i.e. other than a perforated disk (8)); more specifically, flow restrictors similar to those depicted in EP 0552916 A1:
Comp.Ex. 1: Tube 1 was a venturi element resembling that depicted in Fig. 2 of EP 0552916 A1, wherein the proximal inner diameter was taken as 100 % and the venturi section, or stenosis, was proportionally smaller
Comp.Ex. 2: Tube 2 was a venturi element resembling that depicted in Fig. 2 of EP 0552916 A1, wherein the proximal outer diameter was taken as 100 % and the venturin section, or stenosis, was proportionally smaller
Comp.Ex. 3: Tube 3 was a movably arranged `flap-tpye' non-perforated disk resembling that depicted in Fig. 18 of EP 0552916 A1 and exhibiting a similar total surface area, orientation and positioning as the perforated disk (8) in Example 1

The other parts of the spirometer (1), such as the tubular mouthpiece (2) or the main body (9) with the bypass fluid channel (12) and the MEMS-based thermal fluid flow sensor (13, 13.1, 13.2) were kept constant for all four exeperiments (Ex. 1 and Comp.Ex. 1-3).

The results of Comparison Examples 1-3 (Comp.Ex. 1-3) will be provided below.

### Results of Example 1 and Comparison Examples 1-3:

Figures 5A, B, C and D show the steady-flow waveforms measured as a standard procedure of calibration for the four portable spirometers: 64 waveforms starting with 0.15 L/s (left side of x-axis) and increasing gradually to 18 L/s (right side of x-axis) were measured and the resulting raw signals of the MEMS-based thermal fluid flow sensor recorded (y-axis). Fig. 5A shows the steady-flow waveform of a spirometer (1) according to the invention comprising a tubular mouthpiece (2) with a flow restrictor in the form of a perforated disk (8) as described in Example 1. Fig. 5B and 5C show the steady-flow waveform of a spirometer (1) comprising tubular mouthpieces (2) with flow restrictors in the form of the two differently venturi elements as described in Comparison Examples 1 and 2, respectively. Fig. 5D shows the steady-flow waveform of a spirometer (1) comprising a tubular mouthpiece (2) with a movable 'flap-type' flow restrictor as described in Comparison Example 3.

### Calibration results:

As can be seen from these graphs, only the inventive spirometer (1) with the perforated disk (8) as the flow restrictor, yielded reliable calibration results, with both exhalative and inhaltive flow rates producing sensor signals of equal magnitude for any of the tested flow rates in the range of 0.15 to 18 L/s; see e.g. the similar amplitudes in both + / - signal ranges. In contrast, the 'venturi-spirometers' of comparison examples 1 to 2 were not suited for measuring both exhalative and inhalative flow rates because both flows yielded positive signal values, rather than positive or negative, respectively. For the `flap-type-spirometer' of comparison example 3, very specific steady-flow signals were obtained during calibration (with a relatively small difference between lowest and highest flow rate in comparison to other tubes); this is probably due to the moveable flow-restrictor (the'flap') not reliably returning to its original vertical position after during the measured sequence, causing constant changes of the baseline flow resistance inside the mouthpiece (2). Therefore, no reliable flow measurements were possible with the `flap-type-spirometer' of comparison example 3, as becomes apparent from Tables 1 and 2 below.

### Measurement results as of ISO 26782:2009(E) norm and ATS/ERS Standarization:

All numerical results are presented in Tables 1 and 2 below; calculated values displayed in bold do not meet the criteria defined in ISO 26782:2009(E) norm and ATS/ERS Standarization.

As can be seen, only the inventive spirometer (1) with the perforated disk (8) as the flow restrictor meets all criteria for accuracy and repeatability according to ISO 26782:2009(E) norm and ATS/ERS Standarization, while the other spirometers of Comparison examples 1 to 3 using venturi-elements or a movable 'flap' as the flow restrictors fail to meet the criteria defined in the standards repeatedly.

In addition, as mentioned above, only only the inventive spirometer (1) with the perforated disk (8) as the flow restrictor allowed to reliably measure both exhalation- and inhalation flow rates.

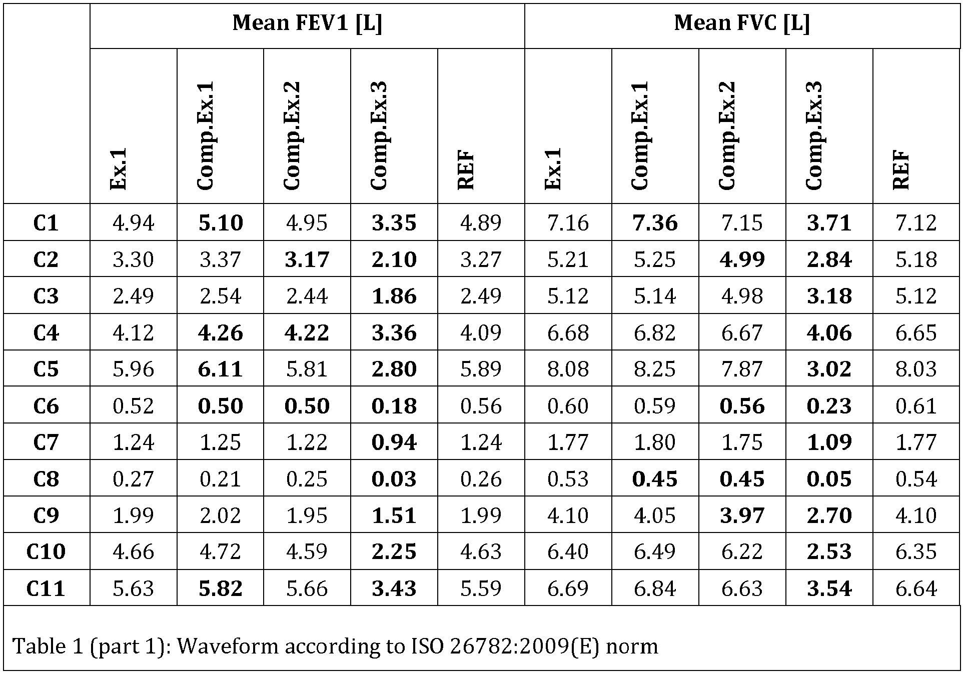

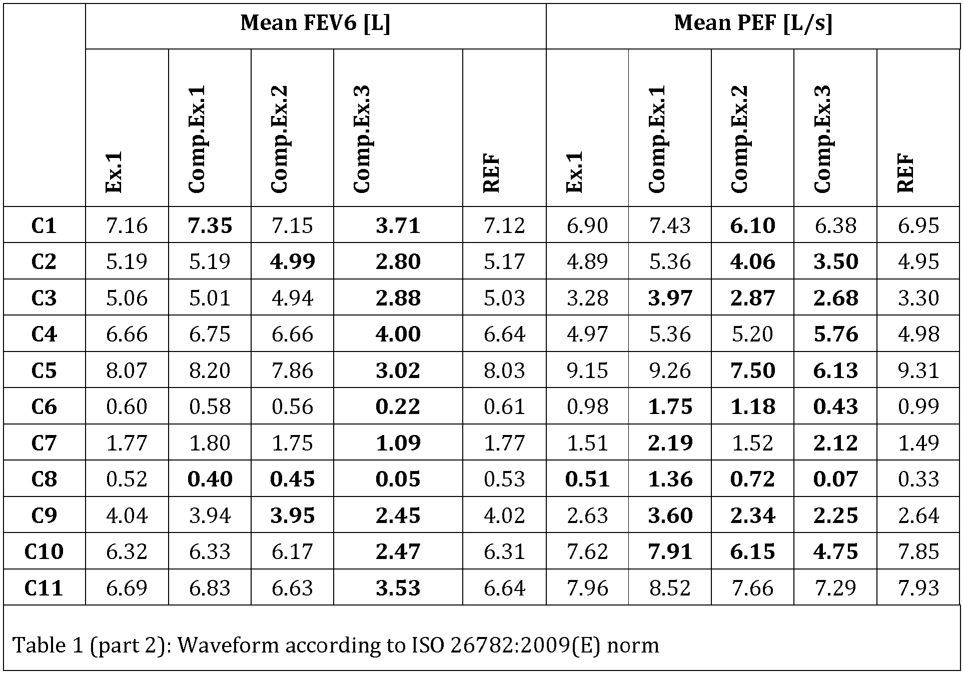

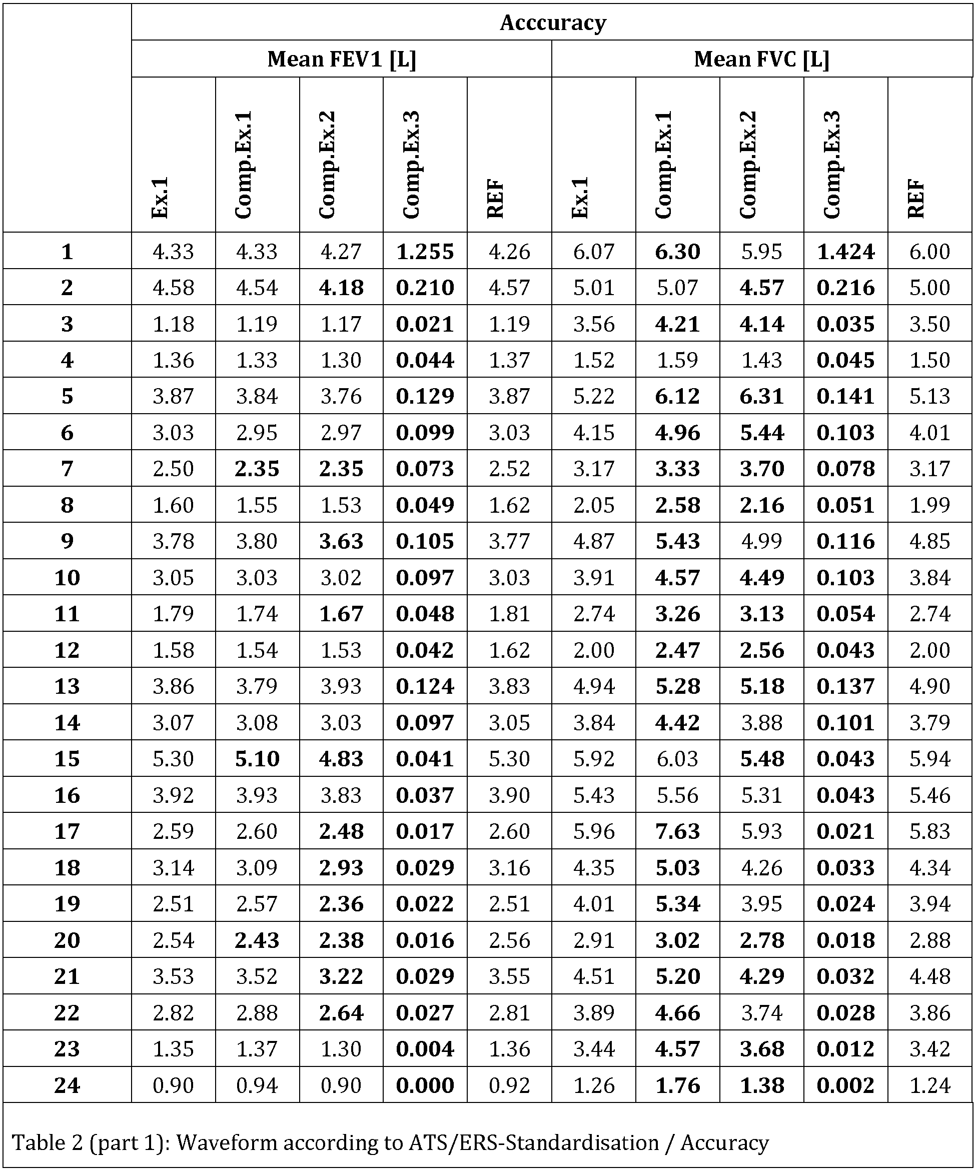

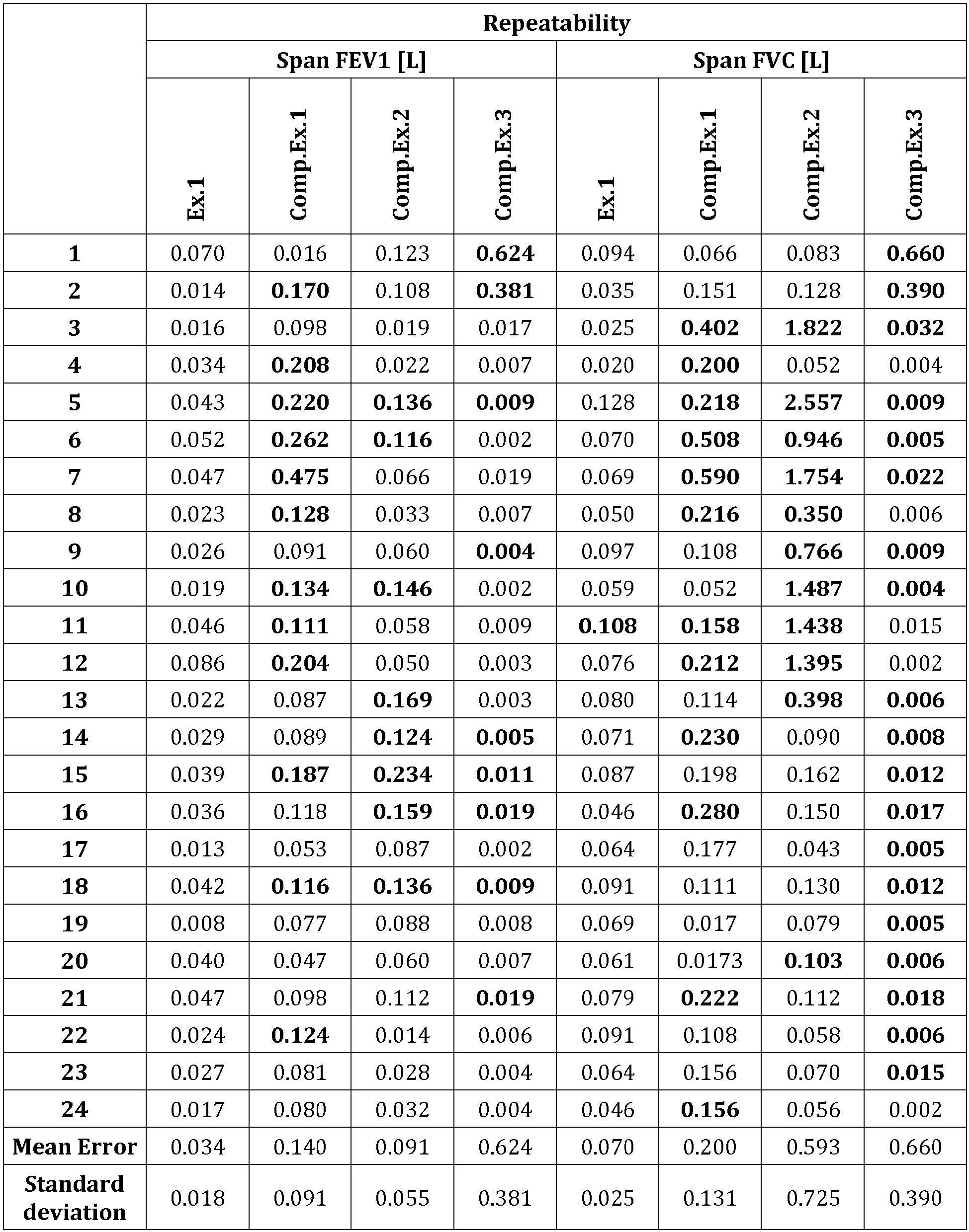

## Claims

1. A portable, handheld electronic spirometer (1) for the evaluation of lung function parameters comprising:
(a) a tubular mouthpiece (2) with
- a proximal opening (3) for insertion into the mouth of a user,
- a distal opening (4),
- a main fluid channel (5) extending between the proximal opening (3) and the distal opening (4) and exhibiting a cross-sectional area (Aₘ) in the range of from about 200 mm² to about 1400 mm²,
- a first lateral opening (6),
- a second lateral opening (7) positioned at a longitudinal distance to the first lateral opening (6), and
- a flow restrictor in the form of a perforated disk (8) positioned in a cross-sectional orientation in the main fluid channel (5) between the first and the second lateral opening (6 and 7); and
(b) a main body (9) with
- a first fluid opening (10) connectible with the first lateral opening (6) of the mouthpiece (2),
- a second fluid opening (11) connectible with the second lateral opening (7) of the mouthpiece (2),
- a bypass fluid channel (12) extending between the first and the second fluid opening (10 and 11) and exhibiting a cross-sectional area (A_{b}) in the range of from about 1 mm² to about 16 mm²,
- a MEMS-based thermal fluid flow sensor (13) positioned at the bypass fluid channel (12) for generating a signal in response to the fluid flow in the bypass fluid channel (12), and
- a microcontroller (14) connected with the fluid flow sensor (13) for calculating the fluid flow from the signal generated by the flow sensor (13) and
- a communication means for the exchange of data related to the fluid flow generated by the micro-controller (14) of the spirometer (1);
wherein
- the cross-sectional area of the main fluid channel (5) ranges from about 250 mm² to about 1300 mm²; and/or the cross-sectional area of the bypass fluid channel (12) ranges from about 1 mm² to about 9 mm²;
- wherein the ratio of the cross-sectional area of the main fluid channel (5) to the cross-sectional area of the bypass fluid channel (12) ranges from about 100 to about 800;
- wherein the total combined area of all perforations (8.1) is from about 26 % to about 72 %, of the cross-sectional area of the main fluid channel (5) at the position of the perforated disk (8), and the perforations (8.1) are optionally circular, or substantially circular, elliptic or polygonal;
- wherein the spirometer (1) exhibits a flow resistance in the range from about 0.01 to about 0.14 kPa/(L/s) at any fluid flow rate within the range of > 0 SLM to about 840 SLM.

2. The spirometer (1) of claim 1, wherein
i) the cross-section of the bypass fluid channel (12) with respect to the cross-section of the main fluid channel (5), and
ii) the perforated disk (8) are adapted or configured in a such way as to cause a fluid flow in the bypass fluid channel (12) which is from about 1 : 2.5 to about 1 : 200 of the fluid flow in the main fluid channel (5); and/or
in a such way as to cause a fluid flow in the bypass fluid channel (12) which ranges from about 0.3 SLM to about 350 SLM.

3. The spirometer (1) of claims 1 or 2, wherein the perforated disk (8) exhibits from about 15 to about 100 perforations (8.1).

4. The spirometer (1) of any one of the preceding claims, wherein the cross-sectional area (Aₘ) of the main fluid channel (5) ranges from about 530 mm² to about 760 mm²; and
wherein the cross-sectional area (A_{b}) of the bypass fluid channel (12) ranges from about 1 mm² to about 4 mm²; and
wherein further the perforated disk (8) exhibits from about 30 to about 60 perforations (8.1) with a circular, or substantially circular, or regular polygonal shape, and a total combined area of all perforations (8.1) from about 30 % to about 50 % of the cross-sectional area of the main fluid channel (5) at the position of the perforated disk (8).

5. The spirometer (1) of any one of the preceding claims, wherein the bypass fluid channel (12) has a rectangular, or substantially rectangular, cross-section.

6. The spirometer (1) of any one of the preceding claims, wherein the MEMS-based thermal fluid flow sensor (13) is arranged centrally on one of the transverse axis of the bypass fluid channel (12) which is perpendicular to one of the longitudinal axis of the bypass fluid channel (12).

7. The spirometer (1) of any one of the preceding claims, wherein the MEMS-based thermal fluid flow sensor (13) is a bidirectional, monolithic CMOS flow sensor (13.1, 13.2) comprising a sensor chip, the chip comprising an encapsulated gas bubble, a microheater in the form of a heating rod comprising a plurality of dynamically controlled thermal resistors for heating the gas bubble, a first plurality of thermopiles arranged in rows and located on a first side of the gas bubble, and a second plurality of thermopiles arranged in rows and located on a second side of the gas bubble which is opposite to the first side.

8. The spirometer (1) of any one of the preceding claims, further comprising an acceleration sensor (15) which is different from the MEMS-based thermal fluid flow sensor (13, 13.1, 13.2).

9. The spirometer (1) of any one of the preceding claims, further comprising a gyroscope (29) which is different from the MEMS-based thermal fluid flow sensor (13, 13.1, 13.2) and the acceleration sensor (15, 15.1).

10. The spirometer (1) of any one of the preceding claims, further comprising one or more of the following sensors:
(a) a heart rate sensor (16),
(b) a blood oxygen saturation sensor (17),
(c) a temperature sensor (18) for measuring the temperature of the environment,
(d) an atmospheric pressure sensor (19),
(e) a moisture sensor (20);
wherein each of the one or more sensors (16-20) is directly or indirectly connected with the microcontroller (14) such that the microcontroller (14) is capable of receiving a signal from each of the one or more sensors.

11. A method for measuring a lung function associated health parameter of a human subject, the method comprising a step of the human subject performing a breathing manoeuvre through the spirometer (1) of any one of the claims 1 to 10.

12. A system, or kit, comprising:
- the portable, handheld electronic spirometer (1) of any one of the claims 1 to 10, and
- a first air quality measurement device comprising communication means adapted for data exchange with the portable, handheld electronic spirometer (1) and/or with a separate computing unit, and equipped with one or more air quality sensors, prefereably air quality sensors selected from the group consisting of humidity sensors, temperature sensors, atmospheric pressure sensors, MOS-type gas sensors ( metal-oxide-semiconductor), airborne-particles sensors, pollen sensors, ozone (O₃) sensors, nitrogen dioxide (NO₂) sensors, sulfur dioxide (SO₂) sensors carbon monoxide (CO) sensors and other type of gas sensors, for determining determine the air quality at the location of the first air quality measurement device, and optionally
- a separate computing unit adapted to collect and analyse at least the data obtained from the spirometer (1) of claims 1 to 10 and from the first air quality measurement device.

## Patentansprüche

1. Tragbares elektronisches Hand-Spirometer (1) zur Evaluierung von Lungenfunktionsparametern, umfassend:
(a) ein röhrenförmiges Mundstück (2) mit
- einer proximalen Öffnung (3) zur Einführung in den Mund eines Benutzers,
- einer distalen Öffnung (4),
- einem Hauptfluidkanal (5), der sich zwischen der proximalen Öffnung (3) und der distalen Öffnung (4) erstreckt und eine Querschnittsfläche (Aₘ) im Bereich von ungefähr 200 mm² bis ungefähr 1400 mm² aufweist,
- einer ersten seitlichen Öffnung (6),
- einer zweiten seitlichen Öffnung (7), die in einem Längsabstand zur ersten seitlichen Öffnung (6) angeordnet ist, und
- einem Durchflussbegrenzer in Form einer perforierten Scheibe (8), die in einer Querschnittsorientierung im Hauptfluidkanal (5) zwischen der ersten und der zweiten seitlichen Öffnung (6 und 7) angeordnet ist; und
(b) einen Hauptkörper (9) mit
- einer ersten Fluidöffnung (10), die mit der ersten seitlichen Öffnung (6) des Mundstücks (2) verbindbar ist,
- einer zweiten Fluidöffnung (11), die mit der zweiten seitlichen Öffnung (7) des Mundstücks (2) verbindbar ist,
- einem Bypassfluidkanal (12), der sich zwischen der ersten und der zweiten Fluidöffnung (10 und 11) erstreckt und eine Querschnittsfläche (A_{b}) im Bereich von ungefähr 1 mm² bis ungefähr 16 mm² aufweist,
- einem MEMS-basierten thermischen Fluiddurchflusssensor (13), der am Bypassfluidkanal (12) zur Erzeugung eines Signals als Reaktion auf die Fluidströmung im Bypassfluidkanal (12) angeordnet ist, und
- einem Mikrocontroller (14), der zur Berechnung der Fluidströmung aus dem vom Durchflusssensor (13) erzeugten Signal mit dem Fluiddurchflusssensor (13) verbunden ist, und
- einem Kommunikationsmittel zum Austauschen von sich auf die Fluidströmung beziehenden Daten, die vom Mikrocontroller (14) des Spirometers (1) erzeugt werden;
wobei
- die Querschnittsfläche des Hauptfluidkanals (5) im Bereich von ungefähr 250 mm² bis ungefähr 1300 mm² liegt; und/oder die Querschnittsfläche des Bypassfluidkanals (12) im Bereich von ungefähr 1 mm² bis ungefähr 9 mm² liegt;
- wobei das Verhältnis der Querschnittsfläche des Hauptfluidkanals (5) zur Querschnittsfläche des Bypassfluidkanals (12) im Bereich von ungefähr 100 bis ungefähr 800 liegt;
- wobei die kombinierte Gesamtfläche aller Perforationen (8.1) ungefähr 26% bis ungefähr 72% der Querschnittsfläche des Hauptfluidkanals (5) an der Position der perforierten Scheibe (8) beträgt, und die Perforationen (8.1) fakultativ kreisförmig oder im Wesentlichen kreisförmig, elliptisch oder polygonal sind;
- wobei das Spirometer (1) einen Durchflusswiderstand im Bereich von ungefähr 0,01 bis ungefähr 0,14 kPa/(L/s) bei einer beliebigen Fluiddurchflussrate im Bereich von >0 SLM bis ungefähr 840 SLM aufweist.

2. Spirometer (1) nach Anspruch 1, wobei
i) der Querschnitt des Bypassfluidkanals (12) bezüglich des Querschnitts des Hauptfluidkanals (5) und
ii) die perforierte Scheibe (8) derart angepasst oder ausgelegt sind, dass sie eine Fluidströmung im Bypassfluidkanal (12) verursachen, die ungefähr 1 : 2,5 bis ungefähr 1 : 200 der Fluidströmung im Hauptfluidkanal (5) beträgt; und/oder derart, dass sie eine Fluidströmung im Bypassfluidkanal (12) verursachen, die im Bereich von ungefähr 0,3 SLM bis ungefähr 350 SLM liegt.

3. Spirometer (1) nach den Ansprüchen 1 oder 2, wobei die perforierte Scheibe (8) ungefähr 15 bis ungefähr 100 Perforationen (8.1) aufweist.

4. Spirometer (1) nach einem der vorhergehenden Ansprüche, wobei die Querschnittsfläche (Aₘ) des Hauptfluidkanals (5) im Bereich von ungefähr 530 mm² bis ungefähr 760 mm² liegt; und
wobei die Querschnittsfläche (A_{b}) des Bypassfluidkanals (12) im Bereich von ungefähr 1 mm² bis ungefähr 4 mm² liegt; und
wobei ferner die perforierte Scheibe (8) ungefähr 30 bis ungefähr 60 Perforationen (8.1) mit einer kreisförmigen oder im Wesentlichen kreisförmigen oder regelmäßigen polygonalen Gestalt aufweist, und eine kombinierte Gesamtfläche aller Perforationen (8.1) ungefähr 30% bis ungefähr 50% der Querschnittsfläche des Hauptfluidkanals (5) an der Position der perforierten Scheibe (8) beträgt.

5. Spirometer (1) nach einem der vorhergehenden Ansprüche, wobei der Bypassfluidkanal (12) einen rechteckigen oder im Wesentlichen rechteckigen Querschnitt aufweist.

6. Spirometer (1) nach einem der vorhergehenden Ansprüche, wobei der MEMSbasierte thermische Fluiddurchflusssensor (13) mittig auf einer der Querachsen des Bypassfluidkanals (12), die senkrecht zu einer der Längsachsen des Bypassfluidkanals (12) ist, angeordnet ist.

7. Spirometer (1) nach einem der vorhergehenden Ansprüche, wobei der MEMSbasierte thermische Fluiddurchflusssensor (13) ein bidirektionaler monolithischer CMOS-Durchflusssensor (13.1, 13.2) ist, der einen Sensorchip umfasst, wobei der Chip eine eingekapselte Gasblase, eine Mikroheizung in Form eines Heizstabs mit einer Vielzahl von dynamisch gesteuerten thermischen Widerständen zum Erwärmen der Gasblase, eine erste Vielzahl von Thermosäulen, die in Reihen angeordnet sind und sich auf einer ersten Seite der Gasblase befinden, und eine zweite Vielzahl von Thermosäulen, die in Reihen angeordnet sind und sich auf einer zweiten Seite der Gasblase, die der ersten Seite gegenüberliegt, befinden, umfasst.

8. Spirometer (1) nach einem der vorhergehenden Ansprüche, ferner umfassend einen Beschleunigungssensor (15), der vom MEMS-basierten thermischen Fluiddurchflusssensor (13, 13.1, 13.2) verschieden ist.

9. Spirometer (1) nach einem der vorhergehenden Ansprüche, ferner umfassend ein Gyroskop (29), das vom MEMS-basierten thermischen Fluiddurchflusssensor (13, 13.1, 13.2) und vom Beschleunigungssensor (15, 15.1) verschieden ist.

10. Spirometer (1) nach einem der vorhergehenden Ansprüche, ferner umfassend einen oder mehrere der folgenden Sensoren:
(a) ein Herzfrequenzsensor (16),
(b) ein Blutsauerstoffsättigungssensor (17),
(c) ein Temperatursensor (18) zur Messung der Temperatur der Umgebung,
(d) ein Atmosphärendrucksensor (19),
(e) ein Feuchtigkeitssensor (20);
wobei jeder der ein oder mehreren Sensoren (16-20) direkt oder indirekt mit dem Mikrocontroller (14) verbunden ist, so dass der Mikrocontroller (14) ein Signal von jedem der ein oder mehreren Sensoren empfangen kann.

11. Verfahren zur Messung eines mit der Lungenfunktion assoziierten Gesundheitsparameters eines menschlichen Individuums, wobei das Verfahren einen Schritt umfasst, in dem das menschliche Individuum ein Atemmanöver durch das Spirometer (1) nach einem der Ansprüche 1 bis 10 durchführt.

12. System, oder Kit, umfassend:
- das tragbare elektronische Hand-Spirometer (1) nach einem der Ansprüche 1 bis 10, und
- eine erste Vorrichtung zur Messung der Luftqualität, umfassend ein Kommunikationsmittel, das für Datenaustausch mit dem tragbaren elektronischen Hand-Spirometer (1) angepasst ist, und/oder mit einer separaten Recheneinheit, und ausgestattet mit einem oder mehreren Luftqualitätssensoren, vorzugsweise Luftqualitätssensoren, die aus der aus Feuchtigkeitssensoren, Temperatursensoren, Atmosphärendrucksensoren, Gassensoren vom MOS-Typ (Metall-Oxid-Halbleiter), Schwebeteilchen-Sensoren, Pollensensoren, Ozon- (Os) Sensoren, Stickstoffdioxid- (NOz) Sensoren, Schwefeldioxid- (SOz) Sensoren, Kohlenmonoxid- (CO) Sensoren und anderen Arten von Gassensoren bestehenden Gruppe ausgewählt sind, zur Bestimmung der Luftqualität am Standort der ersten Vorrichtung zur Messung der Luftqualität, und fakultativ
- eine separate Recheneinheit, die zum Sammeln und Auswerten mindestens der vom Spirometer (1) der Ansprüche 1 bis 10 und von der ersten Vorrichtung zur Messung der Luftqualität erhaltenen Daten ausgelegt ist.

## Revendications

1. Spiromètre électronique portable à main (1) destiné à l'évaluation de paramètres de la fonction pulmonaire et comprenant:
(a) un embout buccal tubulaire (2) pourvu
- d'une ouverture proximale (3) destinée à être insérée dans la bouche d'un utilisateur,
- une ouverture distale (4),
- un conduit de fluide principal (5) qui s'étend entre l'ouverture proximale (3) et l'ouverture distale (4) et qui présente une aire en coupe transversale (Aₘ) dans la gamme allant d'environ 200 mm² à environ 1400mm²,
- une première ouverture latérale (6),
- une deuxième ouverture latérale (7) positionnée à une distance longitudinale de la première ouverture latérale (6), et
- un limiteur de débit se présentant sous la forme d'un disque perforé (8) qui est positionné suivant une orientation en coupe transversale dans le conduit de fluide principal (5) entre la première et la deuxième ouverture latérale (6 et 7) ; et
(b) un corps principal (9) pourvu
- d'une première ouverture de fluide (10) qui peut être raccordée à la première ouverture latérale (6) de l'embout buccal (2),
- d'une deuxième ouverture de fluide (11) qui peut être raccordée à la deuxième ouverture latérale (7) de l'embout buccal (2),
- d'un conduit de fluide de dérivation (12) qui s'étend entre la première et la deuxième ouverture de fluide (10 et 11) et qui présente une aire en coupe transversale (A_{b}) dans la gamme allant d'environ 1 mm² à environ 16 mm²,
- d'un capteur de débit de fluide thermique à base de MEMS (13) qui est positionné au niveau du conduit de fluide de dérivation (12) afin de générer un signal en réponse au débit de fluide dans le conduit de fluide de dérivation (12), et
- d'un microcontrôleur (14) raccordé au capteur de débit de fluide (13) afin de calculer le débit de fluide à partir du signal généré par le capteur de débit (13) et
- d'un moyen de communication destiné à l'échange de données liées au débit de fluide généré par le microcontrôleur (14) du spiromètre (1) ;
- l'aire en coupe transversale du conduit de fluide principal (5) étant dans la gamme allant d'environ 250 mm² à environ 1300 mm² ; et/ou l'aire en coupe transversale du conduit de fluide de dérivation (12) étant dans la gamme allant d'environ 1 mm² à environ 9 mm² ;
- le rapport de l'aire en coupe transversale du conduit de fluide principal (5) à l'aire en coupe transversale du conduit de fluide de dérivation (12) étant dans la gamme allant d'environ 100 à environ 800 ;
- l'aire combinée totale de toutes les perforations (8.1) étant d'environ 26 % à environ 72 % de l'air en coupe transversale du conduit de fluide principal (5) à la position du disque perforé (8), et les perforations (8.1) étant éventuellement circulaires, ou sensiblement circulaires, elliptiques ou polygonales ;
- le spiromètre (1) présentant une résistance à l'écoulement dans la gamme allant d'environ 0,01 à environ 0,14 kPa/(L/s) pour tout débit de fluide dans la gamme de > 0 SLM à environ 840 SLM.

2. Spiromètre (1) selon la revendication 1,
i) la section transversale du conduit de fluide de dérivation (12) par rapport à la section transversale du conduit de fluide principal (5), et
ii) le disque perforé (8) étant adaptés ou conçus de manière à provoquer un débit de fluide dans le conduit de fluide de dérivation (12) qui est d'environ 1:2,5 à environ 1:200 du débit de fluide dans le conduit de fluide principal (5) ; et/ou
de manière à provoquer un débit de fluide dans le conduit de fluide de dérivation (12) qui est dans la gamme d'environ 0,3 SLM à environ 350 SLM.

3. Spiromètre (1) selon les revendications 1 ou 2, le disque perforé (8) comportant environ 15 à environ 100 perforations (8.1).

4. Spiromètre (1) selon l'une quelconque des revendications précédentes, l'aire en coupe transversale (Aₘ) du conduit de fluide principal (5) étant dans la gamme allant d'environ 530 mm² à environ 760 mm² ; et
l'aire en coupe transversale (A_{b}) du conduit de fluide de dérivation (12) étant dans la gamme allant d'environ 1 mm² à environ 4 mm² ; et
en outre le disque perforé (8) comportant environ 30 à environ 60 perforations (8.1) de forme circulaire, ou sensiblement circulaire, ou polygonale régulière, et une aire combinée totale de toutes les perforations (8.1) d'environ 30 % à environ 50 % de l'aire en coupe transversale du conduit de fluide principal (5) à la position du disque perforé (8).

5. Spiromètre (1) selon l'une quelconque des revendications précédentes, le conduit de fluide de dérivation (12) ayant une section transversale rectangulaire ou sensiblement rectangulaire.

6. Spiromètre (1) selon l'une quelconque des revendications précédentes, le capteur de débit de fluide thermique à base de MEMS (13) étant disposé au centre sur l'un des axes transversaux du conduit de fluide de dérivation (12) qui est perpendiculaire à l'un des l'axes longitudinaux du conduit de fluide de dérivation (12).

7. Spiromètre (1) selon l'une quelconque des revendications précédentes, le capteur de débit de fluide thermique à base de MEMS (13) étant un capteur de débit CMOS monolithique bidirectionnel (13.1, 13.2) comprenant une puce de capteur, la puce comprenant un bulle de gaz encapsulé, un micro-radiateur se présentant sous la forme d'une tige chauffante comprenant une pluralité de résistances thermiques à commande dynamique destinées à chauffer la bulle de gaz, une première pluralité de thermopiles disposées en rangées et situées sur un premier côté de la bulle de gaz, et une deuxième pluralité de thermopiles disposées en rangées et situées sur un deuxième côté de la bulle de gaz qui est opposé au premier côté.

8. Spiromètre (1) selon l'une quelconque des revendications précédentes, comprenant en outre un capteur d'accélération (15) qui est différent du capteur de débit de fluide thermique à base de MEMS (13, 13.1, 13.2).

9. Spiromètre (1) selon l'une quelconque des revendications précédentes, comprenant en outre un gyroscope (29) qui est différent du capteur de débit de fluide thermique à base de MEMS (13, 13.1, 13.2) et du capteur d'accélération (15, 15.1).

10. Spiromètre (1) selon l'une quelconque des revendications précédentes, comprenant en outre un ou plusieurs des capteurs suivants :
(a) un capteur de fréquence cardiaque (16),
(b) un capteur de saturation du sang en oxygène (17),
(c) un capteur de température (18) destiné à mesurer la température de l'environnement,
(d) un capteur de pression atmosphérique (19),
(e) un capteur d'humidité (20) ;
chacun des un ou plusieurs capteurs (16 à 20) étant raccordé directement ou indirectement au microcontrôleur (14) de telle sorte que le microcontrôleur (14) soit capable de recevoir un signal de chacun des un ou plusieurs capteurs.

11. Procédé de mesure d'un paramètre de santé associé à la fonction pulmonaire d'un sujet humain, le procédé comprenant une étape dans laquelle le sujet humain effectue une manœuvre respiratoire à travers le spiromètre (1) selon l'une quelconque des revendications 1 à 10.

12. Système, ou kit, comprenant :
- le spiromètre électronique portable à main (1) selon l'une quelconque des revendications 1 à 10, et
- un premier dispositif de mesure de la qualité de l'air comprenant des moyens de communication adaptés à l'échange de données avec le spiromètre électronique portable à main (1) et/ou avec une unité de calcul séparée, et équipé d'un ou plusieurs capteurs de qualité de l'air, de préférence des capteurs de qualité de l'air sélectionnés dans le groupe comprenant des capteurs d'humidité, des capteurs de température, des capteurs de pression atmosphérique, des capteurs de gaz de type MOS (semi-conducteur à oxyde métallique), des capteurs de particules en suspension dans l'air, des capteurs de pollen, de capteurs d'ozone (O₃), des capteurs de dioxyde d'azote (NO2), des capteurs de dioxyde de soufre (SOz), des capteurs de monoxyde de carbone (CO) et d'autres types de capteurs de gaz, pour déterminer la qualité de l'air à l'emplacement du premier dispositif de mesure de la qualité de l'air, et éventuellement
- une unité de calcul distincte adaptée pour collecter et analyser au moins les données obtenues du spiromètre (1) selon les revendications 1 à 10 et du premier dispositif de mesure de la qualité de l'air.
